# EUROPEAN PATENT APPLICATION

(11) **EP 4 659 755 A1**
(43) Date of publication of application: **10.12.2025**
(21) Application number: 24756885.0
(22) Date of filing: 13.02.2024
(51) Int. Cl.: A61K 35/30, A61K 31/436, A61K 31/573, A61K 38/08, A61P 27/02

(54) **THERAPEUTIC DRUG FOR RETINAL PIGMENT EPITHELIUM TEARS**

(30) Priority: 14.02.2023 JP 2023020820
(71) Applicant: RACTHERA Co., Ltd., Tokyo 103-6012 (JP)
(72) Inventor: BANDO Kiyoko, Kobe-shi, Hyogo 650-0047 (JP); MANABE Koichiro, Tokyo 103-6012 (JP); KAMEI Tatsuya, Kobe-shi, Hyogo 650-0047 (JP); TAKAMURA Naoki, Kobe-shi, Hyogo 650-0047 (JP); WATANABE Hitoshi, Kobe-shi, Hyogo 650-0047 (JP)
(74) Representative: Vossius & Partner Patentanwälte Rechtsanwälte mbB
(86) International application number: PCT/JP2024/004888
(87) International publication number: WO 2024/172043

(57) **Abstract**

An object of the present invention is to provide a therapeutic agent for retinal pigment epithelial tears in a human subject. The therapeutic agent for retinal pigment epithelial tears in a human subject of the present invention consists of a suspension of dispersed human retinal pigment epithelial cells.

## Description

### Technical Field

The present invention relates to a therapeutic agent for retinal pigment epithelial tears.

### Background Art

Retinal pigment epithelial (RPE) cells are pigment epithelial cells present in the outermost layer of retina and play an important role, for example, in photoreceptor maintenance, such as phagocytosis of photoreceptor outer segments and recycling of visual substances. Retinal pigment epithelial tears (RPE tears) are known to be pathological conditions related to RPE cells. Retinal pigment epithelial tears are considered to be caused by a rupture in a retinal pigment epithelial cell layer, followed by shrinkage of the retinal pigment epithelial cell layer at the ruptured area or the like, which may lead to visual loss or the like in the long term.

However, there has been no therapeutic agent for retinal pigment epithelial tears. In addition, surgical treatments such as macular translocation and autologous RPE transplantation have been performed in the past, but both methods have high difficulty and invasiveness of surgical procedures and a high risk of complications. Currently, there are no treatment options for retinal pigment epithelial tears, and only follow-up is performed.

On the other hand, transplantation of retinal pigment epithelial cell sheets (RPE sheets) has been considered for age-related macular degeneration and other conditions, but problems have been pointed out with use of RPE sheets, such as the need to make a large retinal incision near the macula, which may cause visual field defects in the same site. Even in minimally invasive subretinal transplantation of a suspension of RPE cells, complications such as inadequate engraftment and epimacular membrane have been reported. Therefore, the formulation and transplantation technique of retinal pigment epithelial cells are still in the process of trial and error and have not yet been completed.

### Citation List

### Non Patent Literature

Non Patent Literature 1: Sugita S et al. "HLA-Matched Allogeneic iPS Cells-Derived RPE Transplantation for Macular Degeneration." J Clin Med. 2020;9(7):2217.

### Summary of Invention

### Technical Problem

An object of the present invention is to provide a therapeutic agent for retinal pigment epithelial tears in a human subject.

### Solution to Problem

As a result of extensive studies to solve the above-described problems of the prior art, the present inventors have found that a suspension of retinal pigment epithelial cells dispersed in a vehicle is useful for the treatment of retinal pigment epithelial tears in a human subject and have thus completed the present invention.

In other words, the invention provides the following:
[1] A therapeutic agent for a retinal pigment epithelial tear in a human subject, consisting of a suspension of dispersed human retinal pigment epithelial cells.
[2] The therapeutic agent according to [1], wherein a viscosity of a vehicle used for dispersing the human retinal pigment epithelial cells is 4 mPa·s or less at a shear rate of 2 (1/s) at 25°C.
[2-1] The therapeutic agent according to [2], wherein the vehicle comprises a pharmaceutically acceptable aqueous liquid.
[2-2] The therapeutic agent according to [2-1], wherein the vehicle comprises a hyaluronic acid.
[2-3] The therapeutic agent according to [2-1] or [2-2], wherein the pharmaceutically acceptable aqueous liquid is a balanced salt solution.
[2-4] The therapeutic agent according to any one of [2-1] to [2-3], wherein the vehicle is free of chondroitin sulfate.
[3] The therapeutic agent according to [1] or [2], wherein a concentration of the human retinal pigment epithelial cells in the suspension is 0.1 × 10⁷ to 1 × 10⁷ cells/mL.
[4] The therapeutic agent according to any one of [1] to [3], wherein a dose volume of the therapeutic agent is 20 to 50 µL.
[5] The therapeutic agent according to any one of [1] to [4], which is administered into a region surrounding the retinal pigment epithelial tear through a port of about 0.4 mm to about 0.65 mm on the sclera of the human subject while forming a bleb.
[6] The therapeutic agent according to [5], wherein the bleb is formed to cover a major axis of the retinal pigment epithelial tear.
[7] The therapeutic agent according to [5] or [6], for use in treatment comprising replacing the vitreous cavity of the human subject with air after administration of the therapeutic agent.
[8] The therapeutic agent according to any one of [2] to [7], wherein after administration of the therapeutic agent to the human subject, no formation of the epiretinal membrane is observed or formation of the epiretinal membrane is reduced to some extent.
[9] The therapeutic agent according to any one of [1] to [8], wherein the retinal pigment epithelial tear is in a state where a retinal pigment epithelial cell layer of the human subject is partially ruptured, and Bruch's membrane exposed without being covered with retinal pigment epithelial cells of the human subject remains as a basement membrane.
[10] The therapeutic agent according to [9], wherein after administration of the therapeutic agent to the human subject, the human retinal pigment epithelial cells become polarized and cover the exposed Bruch's membrane.
[11] The therapeutic agent according to any one of [1] to [10], wherein the human retinal pigment epithelial cells are derived from *in vitro* differentiation of human pluripotent stem cells.
[12] The therapeutic agent according to any one of [1] to [11], wherein the human subject has a human leukocyte antigen (HLA) type that matches with the human retinal pigment epithelial cells.
[13] The therapeutic agent according to any one of [1] to [12], which is administered in combination with at least one immunosuppressive agent.
[14] The therapeutic agent according to [13], wherein the at least one immunosuppressive agent is at least one selected from the group consisting of triamcinolone acetonide, cyclosporine, tacrolimus, prednisolone and dexamethasone.
[15] A method of treatment for a retinal pigment epithelial tear in a human subject, comprising:
   administering to the human subject in need thereof an effective amount of a suspension of dispersed human retinal pigment epithelial cells.
[16] The method of treatment according to [15], comprising administering the suspension into a region surrounding the retinal pigment epithelial tear through a port of about 0.4 mm to about 0.6 mm on the sclera of the human subject to form a bleb.
[17] The method of treatment according to [16], wherein the bleb is formed to cover a major axis of the retinal pigment epithelial tear.
[18] The method of treatment according to any one of [15] to [17], further comprising replacing the vitreous cavity of the human subject with air after administration of the suspension.
[19] The method of treatment according to any one of [15] to [18], wherein the suspension is as defined in any one of [1] to [14].
[20] A cryopreserved formulation comprising retinal pigment epithelial cells, for use as the therapeutic agent according to any one of [1] to [14].
[21] The cryopreserved formulation according to [20], for administration into the human subject as a suspension of the dispersed human retinal pigment epithelial cells, wherein the suspension has been thawed and replaced with a vehicle exhibiting a viscosity of 4 mPa·s or less at a shear rate of 2 (1/s) at 25°C.
[22] Use of a suspension of dispersed human retinal pigment epithelial cells in treatment of a retinal pigment epithelial tear in a human subject.
[23] A suspension of dispersed human retinal pigment epithelial cells, for use in treatment of a retinal pigment epithelial tear in a human subject.
[24] Use of a suspension of dispersed human retinal pigment epithelial cells in production of a therapeutic agent for a retinal pigment epithelial tear in a human subject.

### Advantageous Effects of Invention

The present invention can provide a therapeutic agent for retinal pigment epithelial tears in a human subject. The therapeutic agent can be administered (or transplanted) into the subretinal space of the eyeball of a human subject having a retinal pigment epithelial tear, reducing the burden and side effects on the human subject to engraft at the administration site of the human subject, thereby filling at least a part of the retinal pigment epithelial tear.

### Brief Description of Drawings

[Figure 1] Figure 1 is a schematic view showing a procedure for subretinal administration of RPE cells by a transvitreal method.
[Figure 2] Figure 2 shows fluorescence images of RPE cells subretinally administered to an enucleated porcine eye in Example 1.
[Figure 3] Figure 3 shows low-magnification fluorescent immunostaining images of the retina including a representative administration site in the fluorescent immunostaining of Example 2.
[Figure 4] Figure 4 shows low-magnification fluorescent immunostaining images of the retina including a representative administration site in the fluorescent immunostaining of Example 2.
[Figure 5] Figure 5 shows high-magnification fluorescent immunostaining images of the retina including a representative administration site in the fluorescent immunostaining of Example 2.
[Figure 6A] Figure 6A shows fluorescent immunostaining images of the retina including a representative administration site in the fluorescent immunostaining of Example 3 when a human iPS cell-derived RPE cell suspension was administered using an HBSS(+) vehicle.
[Figure 6B] Figure 6B shows fluorescent immunostaining images of the retina including a representative administration site in the fluorescent immunostaining of Example 3 when a human iPS cell-derived RPE cell suspension was administered using an HBSS(+) vehicle.
[Figure 6C] Figure 6C shows fluorescent immunostaining images of the retina including a representative administration site in the fluorescent immunostaining of Example 3 when a human iPS cell-derived RPE cell suspension was administered using an HBSS(+) vehicle.
[Figure 6D] Figure 6D shows fluorescent immunostaining images of the retina including a representative administration site in the fluorescent immunostaining of Example 3 when a human iPS cell-derived RPE cell suspension was administered using an HBSS(+) vehicle.
[Figure 7A] Figure 7A shows fluorescent immunostaining images of the retina including a representative administration site in the fluorescent immunostaining of Example 3 when a human iPS cell-derived RPE cell suspension was administered using a 100-fold diluted PROVISC vehicle.
[Figure 7B] Figure 7B shows fluorescent immunostaining images of the retina including a representative administration site in the fluorescent immunostaining of Example 3 when a human iPS cell-derived RPE cell suspension was administered using a 100-fold diluted PROVISC vehicle.
[Figure 7C] Figure 7C shows fluorescent immunostaining images of the retina including a representative administration site in the fluorescent immunostaining of Example 3 when a human iPS cell-derived RPE cell suspension was administered using a 100-fold diluted PROVISC vehicle.
[Figure 7D] Figure 7D shows fluorescent immunostaining images of the retina including a representative administration site in the fluorescent immunostaining of Example 3 when a human iPS cell-derived RPE cell suspension was administered using a 100-fold diluted PROVISC vehicle.
[Figure 8] Figure 8 shows the results of an electroretinography (ERG) test performed on RCS rats in Example 6.
[Figure 9] Figure 9 shows the results of a visual evoked potential (VEP) test performed on RCS rats in Example 6.
[Figure 10] Figure 10 shows the results of an optokinetic tracking (OKT) test performed on RCS rats in Example 6.
[Figure 11A] Figure 11A shows representative fluorescent immunostaining images of the RCS rat retina transplanted with human iPS cell-derived RPE cells in Example 6.
[Figure 11B] Figure 11B shows representative fluorescent immunostaining images of the RCS rat retina transplanted with human iPS cell-derived RPE cells in Example 6.
[Figure 11C] Figure 11C shows representative fluorescent immunostaining images of the RCS rat retina transplanted with human iPS cell-derived RPE cells in Example 6.
[Figure 12] Figure 12 shows a representative fluorescent immunostaining image of the RCS rat retina with human iPS cell-derived RPE cells transplanted in Example 6.
[Figure 13] Figure 13 shows representative fluorescent immunostaining images of human iPS cell-derived RPE cells and rat photoreceptor outer segments after transplantation into the RCS rat retina in Example 6.
[Figure 14] Figure 14 shows representative fluorescent immunostaining images of human iPS cell-derived RPE cells and tight junction proteins after transplantation into the RCS rat retina in Example 6.
[Figure 15] Figure 15 shows representative fluorescent immunostaining images of synapses between photoreceptor and bipolar cells present in an outer plexiform layer in Example 6.
[Figure 16] Figure 16 shows the results of evaluating cell adhesion of human iPS cell-derived RPE cells on a laminin-coated substrate in Example 7.
[Figure 17] Figure 17 shows an image of a simulated retinal pigment epithelial tear site formed by scraping a part of the RPE sheet from a plate in Example 8.
[Figure 18] Figure 18 shows the results of evaluating cell adhesion of human iPS cell-derived RPE cells supplemented as a suspension to a simulated retinal pigment epithelial tear site in Example 8.
[Figure 19] Figure 19 shows the results of evaluating cell adhesion of human iPS cell-derived RPE cells supplemented as a suspension to a simulated retinal pigment epithelial tear site in Example 8.
[Figure 20] Figure 20 shows the results of observation of an untreated simulated retinal pigment epithelial tear site in Example 8.
[Figure 21] Figure 21 shows the results of evaluating cell adhesion of human iPS cell-derived RPE cells supplemented as a suspension to a simulated retinal pigment epithelial tear site in Example 8.
[Figure 22] Figure 22 shows the results of evaluating cell adhesion of human iPS cell-derived RPE cells supplemented as a suspension to a simulated retinal pigment epithelial tear site in Example 8.
[Figure 23] Figure 23 shows representative fluorescent immunostaining images of the rat retina in Example 10 when only vehicle was intraperitoneally administered to an F344/NJcl nude rat.
[Figure 24] Figure 24 shows representative fluorescent immunostaining images of the rat retina in Example 10 when sodium iodate was intraperitoneally administered to an F344/NJcl nude rat.
[Figure 25] Figure 25 shows the results of an ERG tests performed on F344/NJcl nude rats in Example 10.
[Figure 26] Figure 26 shows fundus images of F344/NJcl nude rats captured with a fundus camera in Example 10.
[Figure 27] Figure 27 shows representative fluorescent immunostaining of the retina in Example 10 when a vehicle only was administered to a model animal created using an F344/NJcl nude rat.
[Figure 28] Figure 28 shows representative fluorescent immunostaining of the retina in Example 10 in the case of transplantation of human iPS cell-derived RPE cells into a model animal created using an F344/NJcl nude rat.
[Figure 29] Figure 29 shows representative fluorescent immunostaining of the retina in Example 10 in the case of transplantation of human iPS cell-derived RPE cells into a model animal created using an F344/NJcl nude rat.
[Figure 30] Figure 30 shows representative fluorescent immunostaining of the retina in Example 10 when only vehicle was administered to a model animal created using an F344/NJcl nude rat.
[Figure 31] Figure 31 shows representative fluorescent immunostaining of the retina in Example 10 in the case of transplantation of human iPS cell-derived RPE cells into a model animal created using an F344/NJcl nude rat.

### Description of Embodiments

### 1. Definition

As used herein, the term "retinal pigment epithelial (RPE) cell" refers to an epithelial cell present on the outside the neural retina in retina *in vivo.* Whether the cell is a retinal pigment epithelial cell can be easily confirmed by those skilled in the art based on, for example, the expression of cell markers (RPE65, MITF, CRALBP, MERTK, BEST1, TTR, PMEL17 etc.), presence of melanin granules (brown-black), intercellular tight junction, and characteristic polygonal or cobblestone-like cell morphology. Whether the cell has the function of a retinal pigment epithelial cell can be easily confirmed by, for example, the secretory capacity of cytokines such as VEGF and PEDF. In one aspect, the retinal pigment epithelial cells are RPE65-positive cells, MITF-positive cells, or RPE65-positive and MITF-positive cells.

Although RPE cells to be transplanted herein are not particularly limited, it is preferable that the RPE cells be derived from rodents, carnivores, or primates, especially from humans. It is also preferable that the RPE cells be derived from pluripotent stem cells (especially iPS cells), and it is particularly preferable that the RPE cells be derived from *in vitro* differentiation of human pluripotent stem cells (especially iPS cells).

In the present invention, the term "pluripotent stem cell" refers to a cell having self-renewal ability and pluripotency, that is, a stem cell capable of being cultured *in vitro* and having an ability to differentiate into all of cell lineages belonging to three germ layers (ectoderm, mesoderm, endoderm) (pluripotency).

Examples of the pluripotent stem cell include an embryonic stem cell (ES cell) and an induced pluripotent stem cell (iPS cell). Pluripotent stem cells to be used in the present invention are mammalian pluripotent stem cells, preferably pluripotent stem cells of rodents, carnivores, or primates, more preferably human pluripotent stem cells. Examples of the mammals here include primates such as human and monkey, rodents such as mouse, rat, hamster, and guinea pig, carnivores such as canine and cat, and other animals such as swine, bovine, goat, horse, sheep, and rabbit.

Embryonic stem cells can be produced by, for example, culturing an inner cell mass present in the pre-implantation blastocyst stage embryo on a feeder cell or in a medium containing LIF. Specific production methods of the embryonic stem cells are described in, for example WO96/22362, WO02/101057, US5,843,780, US6,200,806, and US6,280,718. The embryonic stem cells are available from given organizations, or a commercially available product can be purchased. For example, human embryonic stem cells, KhES-1, KhES-2 and KhES-3, are available from Institute for Frontier Medical Sciences, Kyoto University. EB5 cell, a mouse embryonic stem cell, is available from RIKEN, and D3 cell line, a mouse embryonic stem cell, is available from ATCC.

Note that the human embryonic stem cells are established from human embryos within 14 days of fertilization.

Nuclear transfer ES cell (ntES cell), one of the ES cells, can be established from a clone embryo produced by transplanting the nucleus of a somatic cell into an enucleated egg.

The term "induced pluripotent stem cell" refers to a cell induced to have pluripotency by reprogramming a somatic cell by a known method. Specific examples thereof include a cell induced to have pluripotency by reprogramming somatic cells such as fibroblasts, skin cells, and peripheral blood mononuclear cells by introduction of any combination of a plurality of reprogramming factors selected from the group consisting of genes such as Oct3/4, Sox2, Klf4, Myc (c-Myc, N-Myc, L-Myc), Glis1, Nanog, Sall4, lin28, and Esrrb. Examples of preferred combinations of the reprogramming factors include (1) Oct3/4, Sox2, Klf4, and Myc (c-Myc or L-Myc), and (2) Oct3/4, Sox2, Klf4, Lin28 and L-Myc (Stem Cells, 2013; 31:458-466).

Induced pluripotent stem cells were first established by Yamanaka et al. in mouse cell in 2006 (Cell, 2006, 126(4), pp. 663-676) and also established in human fibroblast in 2007 (Cell, 2007, 131(5) pp. 861-872; Science, 2007, 318(5858) pp. 1917-1920; Nat. Biotechnol., 2008, 26(1) pp. 101-106). Various improvements have thereafter been made in an induction method of induced pluripotent stem cells, and a specific production method of, for example, a mouse induced pluripotent stem cell is described in Cell. 2006 Aug. 25; 126(4):663-76, and that of a human induced pluripotent stem cell is described in Cell. 2007 Nov. 30; 131(5):861-72.

Besides the production method based on direct reprogramming by gene expression, induced pluripotent stem cells can also be obtained from somatic cell by addition of a compound and the like (Science, 2013, 341, pp. 651-654).

It is also possible to obtain established induced pluripotent stem cells and, for example, human induced pluripotent cell lines established by Kyoto University such as 201B7 cells, 201B7-Ff cells, 253G1 cells, 253G4 cells, 1201C1 cells, 1205D1 cells, 1210B2 cells, and 1231A3 cells are available from Kyoto University and iPS Academia Japan, Inc. As the established induced pluripotent stem cells, for example, Ff-I01 cells, Ff-I14 cells, and QHJI01 cells established by Kyoto University are available from Kyoto University.

The somatic cells used in the production of induced pluripotent stem cells are not particularly limited, and specific examples thereof include fibroblasts and blood-lineage cells (e.g., peripheral blood mononuclear cells or T cells, cord blood-derived cells). Examples of the fibroblasts include those derived from dermis.

When induced pluripotent stem cells are produced through reprogramming by the expression of several reprogramming factors, the means for gene expression is not particularly limited, and a gene transfer method or a direct injection method of protein, which are well known to those skilled in the art, can be used. Specific examples of the gene transfer method include an infection method using a virus vector (e.g., retrovirus vector, lentivirus vector, Sendaivirus vector, adenovirus vector, adeno-associated virus vector), and a calcium phosphate method, lipofection method, RetroNectin method, or electroporation method, each using a plasmid vector (e.g., plasmid vector, episomal vector) or RNA vector.

An induced pluripotent stem cell can be produced in the presence of a feeder cell or absence of feeder cells (feeder-free). When an induced pluripotent stem cell is produced in the presence of a feeder cell, the induced pluripotent stem cell can be produced by a known method in the presence of an undifferentiated maintenance factor. Although a medium to be used for producing an induced pluripotent stem cell in the absence of feeder cells is not particularly limited, a known maintenance medium for embryonic stem cells and/or induced pluripotent stem cells, and a medium for establishing an induced pluripotent stem cell under feeder-free conditions can be used. Examples of the medium for establishing an induced pluripotent stem cell under feeder-free conditions include feeder-free media such as Essential 8 medium (E8 medium), Essential 6 medium, TeSR medium, mTeSR medium, mTeSR-E8 medium, Stabilized Essential 8 medium, and StemFit(R). In the production of an induced pluripotent stem cell, for example, the induced pluripotent stem cell can be produced by gene transfer of 4 factors of Oct3/4, Sox2, Klf4, and Myc into a somatic cell by using a Sendaivirus vector in the absence of feeder cells.

The pluripotent stem cells to be used in the present invention are preferably induced pluripotent stem cells of rodents, carnivores, or primates, more preferably human induced pluripotent stem cells.

Although those skilled in the art can perform maintenance culture or expansion culture of pluripotent stem cells by a well-known method, it is preferable that the pluripotent stem cells be subjected to maintenance culture or expansion culture under serum-free conditions and in the absence of feeder cells from the viewpoint of the safety of graft cell production, for example.

Genetically-modified pluripotent stem cells can be produced by using, for example, a homologous recombination technique. Examples of the gene on the chromosome to be modified include a cell marker gene, a histocompatibility antigen gene, a gene related to a disease based on a disorder of retinal pigment epithelial cells. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); and Biomanual Series 8, Gene Targeting, Making of Mutant Mouse using ES cell, YODOSHA CO., LTD. (1995).

Specifically, for example, the genomic DNA comprising the target gene to be modified (e.g., cell marker gene, histocompatibility antigen gene, disease-related gene) is isolated, and a targeting vector for homologous recombination of the target gene is produced using the isolated genomic DNA. After the produced targeting vector is introduced into stem cells, the cells in which homologous recombination has occurred between the target gene and the targeting vector are selected, whereby stem cells having the modified gene on the chromosome can be produced.

Examples of a method of isolating genomic DNA comprising the target gene include known methods described in Molecular Cloning, A Laboratory Manual, Second Edition, Cold Spring Harbor Laboratory Press (1989) and Current Protocols in Molecular Biology, John Wiley & Sons (1987-1997). The genomic DNA comprising the target gene can also be isolated using genomic DNA library screening system (manufactured by Genome Systems, Inc.) and Universal GenomeWalker Kits (manufactured by CLONTECH Laboratories, Inc.), for example. A polynucleotide encoding the target protein can also be used instead of genomic DNA. The polynucleotide can be obtained by amplifying the corresponding polynucleotide by the PCR method.

Production of targeting vector for homologous recombination of the target gene and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995), for example. As the targeting vector, any of replacement type or insertion type can be used. As the selection method, methods such as positive selection, promoter selection, negative selection, and polyA selection can be used.

Examples of a method of selecting the desired homologous recombinant from the selected cell lines include Southern hybridization method and PCR method for the genomic DNA.

The method for producing a retinal pigment epithelial cell from a pluripotent stem cell is not particularly limited as long as it is a known method, and may be, for example, the methods described in WO2005/070011, WO2006/080952, WO2009/051671, WO2011/063005, WO2012/173207, WO2015/053375, WO2014/087244, WO2014/121077, WO2015/053376, WO2015/068505, WO2017/043605, Stem Cell Reports, 2(2), 205-218 (2014), and Cell Stem Cell, 10(6), 771-785 (2012). For example, the method may be a production method of a retinal pigment epithelial cell, including the following steps (1) and (2) disclosed in WO2017/043605:
(1) a first step of culturing a pluripotent stem cell in a medium containing an FGF receptor inhibitor and/or an MEK inhibitor for a period of not more than 30 days (preferably 2 days to 13 days or 2 days to 6 days, more preferably 4 days to 6 days), and
(2) a second step of culturing the cell obtained in the first step in the presence of a Nodal signal transduction pathway inhibitor and/or a Wnt signal transduction pathway inhibitor to form a retinal pigment epithelial cell.

The production method of a retinal pigment epithelial cell may also include the following steps (1) and (2):
(1) a first step of culturing a pluripotent stem cell in a medium containing an FGF receptor inhibitor and/or an MEK inhibitor for a period sufficient for inducing gene expression of at least one eye field transcription factor and of not more than 30 days, and
(2) a second step of culturing the cell obtained in the first step in the presence of a Nodal signal transduction pathway inhibitor and/or a Wnt signal transduction pathway inhibitor to form a retinal pigment epithelial cell.

The production method of a retinal pigment epithelial cell may further include the following steps (1) and (2):
(1) a first step of culturing a pluripotent stem cell in a medium containing an FGF receptor inhibitor and/or an MEK inhibitor for a period sufficient for inducing gene expression of at least one of PAX6, LHX2, and SIX3 and of not more than 30 days, and
(2) a second step of culturing the cell obtained in the first step in the presence of a Nodal signal transduction pathway inhibitor and/or a Wnt signal transduction pathway inhibitor to form a retinal pigment epithelial cell.

The FGF receptor inhibitor is not particularly limited as long as it is a substance capable of suppressing signal transduction mediated by FGF, and may be any of proteins, nucleic acids, and low-molecular-weight compounds. Herein, FGF forms a family consisting of at least 22 species. Example of the representative FGF receptor include FGFR1, FGFR2, FGFR3, and FGFR4, and the FGF receptor inhibitor is a substance that inhibits one, multiple, or all of them. Examples of the substance include, but are not limited to, a substance that directly acts on FGF or FGF receptors (e.g., antibody, aptamer), a substance that suppresses expression of a gene encoding FGF or FGF receptors (e.g., antisense oligonucleotide, siRNA), a substance that inhibits binding of FGF receptors to FGF (e.g., soluble FGF receptor, FGF antagonist), and a substance that inhibits physiological activity caused by signal transduction by FGF receptors, such as a low-molecular-weight compound including PD173074 (N-[2-[[4-(diethylamino)butyl]amino]-6-(3,5-dimethoxyphenyl)pyrido[2,3-d]pyrimidin-7-yl]-N'-(1,1-dimethylethyl)urea) or SU5402 (2-[(1,2-dihydro-2-oxo-3H-indol-3-ylidene)methyl]-4-methyl-1H-pyrrole-3-propanoic acid) that inhibits tyrosine kinase activity of FGF receptors by ATP competition. Only one kind of the substance may be used, or two or more kinds thereof may be used in combination. Here, PD173074 and SU5402 are known FGF receptor inhibitors, and a commercially available product and the like can be obtained as appropriate. Preferred examples of the FGF receptor inhibitors include PD173074 and SU5402.

The MEK inhibitor is not particularly limited as long as it is a substance that inhibits expression or activity of MEK family, and may be any of proteins, nucleic acids, and low-molecular-weight compounds. Example of the representative MEK family include MEK1, MEK2, and MEK3, and the MEK inhibitor is a substance that inhibits the expression or activity of one, multiple, or all of these MEK families. Examples of the substance include, but are not limited to, a substance that suppresses expression of a gene encoding various MEK (e.g., antisense oligonucleotide, siRNA), a substance that inhibits enzyme activity of various MEK, such as a low-molecular-weight compound including PD0325901 (N-[(2R)-2,3-dihydroxypropoxy]-3,4-difluoro-2-[(2-fluoro-4-iodophenyl)amino]-benzamide), PD184352 ((2-[(2-chloro-4-iodophenyl)amino]-N-cyclopropylmethoxy)-3,4-difluorobenzamide), PD98059 (2'-amino-3'-methoxyflavone), U0126(1,4-diamino-2,3-dicyano-1,4-bis[2-aminophenylthio]butadiene), MEK162 (5-[(4-bromo-2-fluorophenyl)amino]-4-fluoro-N-(2-hydroxyethoxy)-1-methyl-1H-benzimidazole-6-carboxamide), SL327(α-[amino[(4-aminophenyl)thio]methylene]-2-(trifluoromethyl)benzeneacetonitrile), TAK-733 ((R)-3-(2,3-dihydroxypropyl)-6-fluoro-5-(2-fluoro-4-iodophenylamino)-8-methylpyrido [2,3-d] pyrimidine-4,7 (3H,8H)-dione), or AZD-8330 (2-(2-fluoro-4-iodophenylamino)-N-(2-hydroxyethoxy)-1,5-dimethyl-6-oxo-1,6-dihydropyridine-3-carboxamide). One kind of the substance may be used, or two or more kinds thereof may be used in combination. Here, PD0325901, PD184352, PD98059, U0126, MEK162, SL327, TAK-733, and AZD-8330 are known MEK inhibitors, and a commercially available product and the like can be obtained as appropriate. Preferred examples of the MEK inhibitors include PD0325901, PD184352, U0126, TAK-733, and AZD-8330.

The Nodal signal transduction pathway inhibitor is not particularly limited as long as it can suppress signal transduction mediated by Nodal, and may be any of proteins, nucleic acids, and low-molecular-weight compounds. The signal mediated by Nodal is transduced via a Nodal receptor. The Nodal receptor is present as a heterodimer of TYPE I receptor (ALK (activin receptor-like kinase)-4, ALK-5, ALK-7) and TYPE II receptor (ActRII). Examples of the Nodal signal transduction pathway inhibitor include, but are not limited to, a substance that directly acts on Nodal or Nodal receptors (anti-Nodal antibody, anti-Nodal receptor antibody etc.), a substance that suppresses expression of a gene encoding Nodal or Nodal receptors (e.g., antisense oligonucleotide, siRNA etc.), a substance that inhibits binding of Nodal receptor to Nodal (Lefty-A, Lefty-B, Lefty-1, Lefty-2, soluble Nodal receptor etc.), and a substance that inhibits physiological activity caused by signal transduction by Nodal receptors, such as low-molecular-weight compounds including SB-431542 (SB431542) (4-[4-(1,3-benzodioxol-5-yl)-5-(pyridin-2-yl)-1H-imidazol-2-yl]benzamide) that inhibits kinase activity of TYPE I receptor by ATP competition. SB-431542 is a known Nodal signal inhibitor, and a commercially available product and the like can be obtained as appropriate. Preferred examples of the Nodal signal inhibitor include SB-431542.

The Wnt signal transduction pathway inhibitor is not particularly limited as long as it can suppress signal transduction mediated by Wnt, and may be any of proteins, nucleic acids, and low-molecular-weight compounds. The signal mediated by Wnt is transduced via a Wnt receptor present as a heterodimer of Frizzled (Fz) and LRP5/6 (low-density lipoprotein receptor-related protein 5/6). Examples of the Wnt signal transduction pathway inhibitor include, but are not limited to, a substance that directly acts on Wnt or Wnt receptors (anti-Wnt antibody, anti-Wnt receptor antibody etc.), a substance that suppresses expression of a gene encoding Wnt or Wnt receptors (e.g., antisense oligonucleotide, siRNA), a substance that inhibits binding of Wnt receptors to Wnt (soluble Wnt receptor, dominant negative Wnt receptor or the like, Wnt antagonist, Dkk1, Cerberus protein etc.), and a substance that inhibits physiological activity caused by signal transduction by Wnt receptor, such as low-molecular-weight compounds including casein kinase I inhibitors CKI-7 (N-(2-aminoethyl)-5-chloroisoquinoline-8-sulfonamide) and D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide) that stabilizes β-catenin destruction complex by inhibiting metabolic turnover of Axin, and IWP-2 (N-(6-methyl-2-benzothiazolyl)-2-[(3,4,6,7-tetrahydro-4-oxo-3-phenylthieno[3,2-d]pyrimidin-2-yl)thio]acetamide) that inactivates Porcupine (Porcn) as a membrane-bound O-acyltransferase (MBOAT) and suppresses palmitoylation of Wnt protein. CKI-7, D4476, IWR-1-endo (IWR1e), IWP-2 and the like are known Wnt signal inhibitors, and a commercially available product and the like can be obtained as appropriate. Preferred examples of the Wnt signal inhibitor include CKI-7.

As used herein, the term "eye field transcription factor" refers to a gene expressed in the eye field region in an early developmental stage, and for example, ET(Tbx3), Rx1(Rax), Pax6, Six3, Lhx2, Tlx(Nr2e1), and Optx2(Six6) have been identified. These eye field transcription factors can be used as markers of early eye formation stage.

The number of days of the first step is not particularly limited as long as it is within the period when the cells generated by exposure of pluripotent stem cells to, for example, the FGF receptor inhibitor and/or MEK inhibitor, maintain differentiation potency into retinal pigment epithelial cells, and the pluripotent stem cells in the first step are cultured for a period of not more than 30 days. The period may vary according to the line of the pluripotent stem cells to be used and the like, but is generally 2 days or more, preferably 3 days or more, more preferably 4 days or more. In the first step, the pluripotent stem cells are still more preferably cultured for 2 days to 13 days or 2 days to 6 days, further preferably 4 days to 6 days.

A culture period of the second step is not particularly limited as long as it is a period capable of inducing retinal pigment epithelial cells of interest, but retinal pigment epithelial cells can be produced, as an example of such culture period, generally on days 14 to 40 calculating from the start of the second step. Those skilled in the art can confirm the generation of retinal pigment epithelial cells based on, for example, the expression of a cell marker (RPE65(retinal pigment epithelial cell), Mitf (retinal pigment epithelial cell), BEST1 (retinal pigment epithelial cell), CRALBP (retinal pigment epithelial cell) etc.), presence of melanin granules (brown-black), intercellular tight junction, and characteristic polygonal or cobblestone-like cell morphology, and determine the culture period by confirming them.

After the completion of the second step, it is preferable to exchange the medium with a maintenance medium for retinal pigment epithelial cells (hereinafter, also referred to as an RPE maintenance medium) and further culture the same. For example, the cells are cultured for about 5 to 20 days while exchanging the total amount of the medium at a frequency of at least once every 3 days. As the maintenance medium for retinal pigment epithelial cells, for example, those described in IOVS, March 2004, Vol. 45, No. 3, Masatoshi Haruta, et. al., IOVS, November 2011, Vol. 52, No. 12, Okamoto and Takahashi, J. Cell Science 122 (17), Fumitaka Osakada, et. al., IOVS, February 2008, Vol. 49, No. 2, Gamm, et. al. can be used, which are composed of a basal medium, a serum, and/or a serum replacement, and other components.

The retinal pigment epithelial cells can also be produced by omitting the first step and conducting only the second step.

When retinal pigment epithelial cells are produced by adhesion culture, the retinal pigment epithelial cells can be adhered to each other to form a sheet-like structure. Therefore, a sheet of retinal pigment epithelial cells can be produced. The sheet of retinal pigment epithelial cells may be dissociated into individual cells (single cells) using methods known to those skilled in the art, such as treatment with enzymes including trypsin. The resulting cells may then be suspended in a dispersion vehicle to prepare a suspension suitable for transplantation into a patient.

The term "adhesion culturing" refers to culturing under conditions in which a cell or cell aggregate is adhered to a culture vessel material or the like. In this case, adhesion of cells means that a strong cell-substrate binding is formed between a cell or cell aggregate and a culture vessel material. In other words, adhesion culturing refers to culturing under conditions in which a strong cell-substrate binding is formed between a cell or cell aggregate and a culture vessel material or the like. Those skilled in the art can appropriately set the culturing conditions such as a culture vessel material to conduct adhesion culturing.

The retinal pigment epithelial cells can also be produced by suspension culturing. In other words, an embryoid body (also referred to as EB) can be produced from pluripotent stem cells and induced to differentiate into retinal pigment epithelial cells to produce a cell aggregate containing retinal pigment epithelial cells. The cell aggregate may be dissociated into individual cells (single cells) using methods known to those skilled in the art, such as treatment with enzymes including trypsin. The resulting cells may then be suspended in a dispersion vehicle to prepare a suspension suitable for transplantation into a patient.

After dispersing the cell aggregates/sheets of retinal pigment epithelial cells produced by the above-described methods, the retinal pigment epithelial cells can be suspended in a freezing vehicle and frozen to prepare a cryopreserved formulation containing retinal pigment epithelial cells that can be stored for a long period of time.

The freezing medium used here is not particularly limited as long as it does not adversely affect the function or survival of the retinal pigment epithelial cells, and may be any medium containing a cryoprotectant. The cryoprotectant may be any substance effective in protecting cells and organelles (e.g., capable of preventing ice crystals from forming within cells when the cells are frozen), and examples thereof include dimethyl sulfoxide (DMSO), glycerol, polyethylene glycol, propylene glycol, glycerin, sorbitol, dextran, and trehalose. A commercially available cell cryopreservation solution (e.g., StemCellBanker (Zenoaq)) may also be used as a freezing medium containing a cryoprotectant.

A suspension that can be transplanted into a patient can be prepared by thawing a cryopreserved formulation containing retinal pigment epithelial cells and replacing or suspending it with a dispersion vehicle.

As used herein, the term "retinal pigment epithelial tear" refers to a condition in which a tear occurs in the retinal pigment epithelium cell layer in retina *in vivo* and a pathological condition having partial defect or loss of the retinal pigment epithelium cell layer. The retinal pigment epithelium tear is a pathological condition in which the retinal pigment epithelium cell layer is ruptured, contracts and rolls from the rupture, and partially lacks the retinal pigment epithelium. It also exposes the Bruch's membrane and choroid beneath the retinal pigment epithelium cell layer. The most classic shape of a retinal pigment epithelium tear is a crescent-shaped tear, but there are various shapes, such as a concentric tear with an island of rolled-up retinal pigment epithelium in the center of the tear.

Therefore, in the present specification, the cause of the "retinal pigment epithelium tear" is not particularly limited, and it includes both retinal pigment epithelium tears caused by retina-, choroid-, or other eye-related diseases, and retinal pigment epithelium tears that are not caused by retina-related diseases such as trauma.

When a retinal pigment epithelium tear occurs, the layered structure of the retinal pigment epithelium and photoreceptors is destroyed, resulting in a breakdown of the visual cycle and a loss of the dark curtain effect of the retinal pigment epithelium in the short term, and in the long term in damage to or degeneration of the photoreceptors. The size of the tear correlates with the decline in visual function, and if the tear overlaps with the fovea, it often leads to severe decline in visual function. In addition, if the tear occurs near the fovea, it causes a paracentral scotoma, resulting in severe visual impairment.

A retinal pigment epithelial tear is often associated with age-related macular degeneration (AMD). The retinal pigment epithelial tear may also occur in patients with a pigment epithelial detachment (PED). Note that PED is a dome-like elevation of the retinal pigment epithelium detached from Bruch's membrane, with a space in which serous fluid, blood, fibrovascular membrane, drusenoid material, or a combination thereof is stored.

The retinal pigment epithelial tear occurs when a tear forms in the periphery of a pigment epithelial detachment (PED) especially caused by choroidal neovascularization (CNV), as a result of natural progression, after photodynamic therapy, or after intravitreal injection of an anti-vascular endothelial growth factor (VEGF) drug. It has also been reported that the incidence of retinal pigment epithelial tears in patients with exudative age-related macular degeneration accompanied by a pigment epithelial detachment is 10 to 12%.

Diagnosis of retinal pigment epithelial tears is made by various imaging modalities. For example, optical coherence tomography (OCT) is one of the best methods to confirm retinal pigment epithelial tears because it can clearly identify a defect or rolled-up region of the retinal pigment epithelial. In the fundus findings of retinal pigment epithelial tears, the torn part of the retinal pigment epithelium rolls to the edge of the tear, forming a brown raised lesion. The defective site of the retinal pigment epithelium is slightly depressed, and the choroidal vessels are more clearly visible than in normal areas. In fluorescein angiography, a site where the rolled retinal pigment epithelium is piled up has hypo fluorescence because the fluorescence from the choroid is blocked. In the defective site of the retinal pigment epithelium, strong hyperfluorescence from the choriocapillaris tissue is seen. In fundus autofluorescence, the defective site of the retinal pigment epithelium has hypofluorescence, and the rolled site has hyperfluorescence in the early stage of onset.

### 2. Therapeutic Agent

The therapeutic agent of the present invention is a therapeutic agent for retinal pigment epithelial tears in a human subject and consists of a suspension of dispersed human retinal pigment epithelial cells in a dispersion vehicle. The therapeutic agent of the present invention can be used for the treatment of retinal pigment epithelial tears in a human subject by administrating (or transplanting) human retinal pigment epithelial cells into the subretinal space of the eyeball.

By administrating a suspension of retinal pigment epithelial cells to the lesion of a retinal pigment epithelial tear, it is expected to reduce the risk of complications or the like, in comparison with surgical procedures and the like that have been previously performed, to reconstruct the structure in which the retinal pigment epithelium with normal function and the photoreceptor layer are in contact with each other, and to restore or maintain the visual function (visual acuity, retinal sensitivity, etc.), or to delay or inhibit the deterioration of the visual function. Further effects of using the therapeutic agent of the present invention will be described in detail below.

The human retinal pigment epithelial cells in the therapeutic agent of the present invention may be the human retinal pigment epithelial cells described in 1. Definition, and more preferably are derived from *in vitro* differentiation of human pluripotent stem cells (especially human iPS cells). The retinal pigment epithelial cells may be dispersed retinal pigment epithelial cells, for example, dispersed cells obtained by harvesting a sheet of retinal pigment epithelial cells and dissociating it into single cells and/or cell clusters of 2 to 5 cells.

It is preferable that in the therapeutic agent, the human retinal pigment epithelial cells are contained at a concentration of 0.1 × 10⁷ cells/mL to 1 × 10⁷ cells/mL, and it is more preferable that the concentration is 0.5 × 10⁷ cells/mL to 1 × 10⁷ cells/mL. The dose volume of the therapeutic agent may be 20 µL to 80 µL, and it is preferable that the dose volume be 20 µL to 50 µL, and more preferable that the dose volume be 30 µL to 50 µL. In other words, the dose may be, for example, 0.2 × 10⁵ cells to 5 × 10⁵ cells, or 1 × 10⁵ cells to 5 × 10⁵ cells.

At a human retinal pigment epithelial cell concentration exceeding 1 × 10⁷ cells/mL, the visibility around the injection point is locally deteriorated upon subretinal injection, and there is a risk of damaging the retina during administration, and therefore, the concentration may be set as an upper limit. In order to maximize the efficacy of the therapeutic agent of the present invention in the treatment of retinal pigment epithelial tears, it is required to reconstruct the structure in which the photoreceptor layer of the lesion and the administered retinal pigment epithelial cells are in contact with each other over as wide an area as possible. From the results of transplantation into cynomolgus monkeys, which have a macula, and whose eyeball size and anatomical characteristics are similar to humans, it has been found that the diameter of the bleb formed in the retina of cynomolgus monkeys by administration of 50 µL is sufficient in volume to cover an average retinal pigment epithelial tear lesion (e.g., 1 to 15 mm² or 3 to 10 mm²) or a human fovea and parafovea (diameter: about 2 to 4 mm (e.g., 3 mm), area: about 6 to 8 mm² (e.g., 7.07 mm²)). Since up to 70 to 80 µL per bleb can be subretinally administered to cynomolgus monkeys with good reproducibility, the dose volume described above can be set.

When the therapeutic agent of the present invention is administered into cynomolgus monkeys at the above-described human retinal pigment epithelial cell concentration and dose volume, the cells can be engrafted over an area of, for example, about 3 to 5 mm wide. Therefore, compared to the case of transplanting a sheet-like retinal pigment epithelial cell, the case of transplanting a retinal pigment epithelial cell in a suspension can more easily cover a wider range of pathological sites and also reduce the burden of surgery. Furthermore, when human retinal pigment epithelial cells are administered as a suspension, they can be engrafted at a site where a retinal pigment epithelial tear is present and transplantation is required in the retina of the administration subject. Therefore, the treatment is adaptable to any tear morphologies.

When a sheet of retinal pigment epithelial cells is to be transplanted, a retinotomy is required to insert the sheet, which is at least as wide as the sheet or the device used to insert the sheet. Retinotomy is an iatrogenic retinal tear, which can be said to artificially create a tear in the retina. In addition, when the incision wound is large, the risk of retinal detachment after surgery or the like increases. To reduce the risk of postoperative retinal detachment, it may be necessary to perform photocoagulation around the incision wound. However, such treatment may cause irreversible damage to the normal retina and the normal retinal pigment epithelial cell layer, potentially affecting visual function. In cases of retinal pigment epithelial tears, the area surrounding the lesion generally retains normal visual function. Therefore, photocoagulation around the administration site is considered inappropriate because it may reduce visual function in the normal site more than recovery achieved by treatment.

On the other hand, in the administration of the suspension of dispersed human retinal pigment epithelial cells of the present invention, an administration method or the like described below can be employed, and for example, the administration can be performed only by creating an injection point having a size of about the tip of a subretinal injection needle with an outer diameter of 38 gauge in the retina. In the present invention, these advantages of the suspension of human retinal pigment epithelial cells have been confirmed for the first time, and it has been found that the suspension can be administered to retinal pigment epithelial tears that may have various shapes. Note that these can be optimized by combining with a vehicle and administration method described below.

The vehicle used for dispersion of human retinal pigment epithelial cells in the therapeutic agent of the present invention (hereinafter, also referred to as "dispersion vehicle") is not particularly limited, and the upper limit of the viscosity at a shear rate of 2 (1/s) at 25°C may be 4 mPa·s or less, 3.5 mPa·s or less, or 3 mPa·s or less. The lower limit of the viscosity at a shear rate of 2 (1/s) at 25°C is not particularly limited, but may be, for example, 0.5 mPa·s or more, 0.6 mPa·s or more, 0.7 mPa·s or more, or 0.8 mPa·s or more.

By using the dispersion vehicle in the predetermined viscosity range, it is possible to particularly suppress prolonged retinal detachment upon subretinal administration of retinal pigment epithelial cells, thereby reducing side effects such as formation of epiretinal membrane and retinal detachment, as well as improving engraftment of retinal pigment epithelial cells at the administration site. In the pathological condition of retinal pigment epithelial tears, the above can be set as the viscosity to particularly prevent prolonged retinal detachment because the continuation of retinal detachment may worsen the pathological condition by extending the period during which the retinal pigment epithelial layer does not exert a direct effect on the photoreceptor occurs.

In one aspect of the dispersion vehicle, the viscosity at a shear rate of 2 (1/s) at 25°C may be 1.3 to 3.8 mPa·s, 1.5 to 3.5 mPa·s, or 2 to 3 mPa·s.

The dispersion vehicle may contain a pharmaceutically acceptable aqueous liquid or may be a pharmaceutically acceptable aqueous liquid. The "pharmaceutically acceptable aqueous liquid" is not particularly limited as long as it is an aqueous solution that can be administered into the body and has physical properties suitable for transplantation, and may be, for example, an aqueous solution containing a buffer, a transfusion, saline, injectable water, or perfusate. Preferred examples thereof include a buffer. The dispersion vehicle may contain, in addition to the pharmaceutically acceptable aqueous liquid, a substance that affects viscosity (a substance added for the purpose of increasing viscosity, e.g., a thickener). As described above, examples of the substance that affects viscosity include hyaluronic acid and chondroitin sulfate, and other substances that affect the viscosity (e.g., a thickener) are preferably not included.

In one aspect, the dispersion vehicle may contain hyaluronic acid. The dispersion vehicle may contain chondroitin sulfate, but is preferably free of chondroitin sulfate. Both hyaluronic acid and chondroitin sulfate are mucopolysaccharides present in the body and function as thickening components of the dispersion vehicle.

In the present specification, hyaluronic acid is a linear polymeric polysaccharide with a molecular weight of tens of thousands to millions, in which the linear polymeric polysaccharide having a structure in which D-glucuronic acid and D-N-acetylglucosamine are linked alternately by β-1,4 and β-1,3 glycosidic bonds. Hyaluronic acid has high water-holding capacity and viscosity and is a substance widely used in, for example, pharmaceuticals and cosmetics. As used herein, the term "hyaluronic acid" refers to a concept including both free hyaluronic acid and a salt thereof. Examples of the salt of hyaluronic acid include alkali metal salts and alkaline earth metal salts of hyaluronic acid, and specifically, commercially available sodium hyaluronate, potassium hyaluronate, and the like can be used.

Chondroitin sulfate is mucopolysaccharide with a structure in which sulfuric acid is bound to a sugar chain composed of two repeating saccharides D-glucuronic acid (GlcA) and N-acetyl-D-galactosamine (GalNAc). As used herein, the term "chondroitin sulfate" refers to a concept including both free chondroitin sulfate and a salt thereof. Examples of the salt of chondroitin sulfate include alkali metal salts and alkaline earth metal salts of chondroitin sulfate, and specifically, commercially available sodium chondroitin sulfate, potassium chondroitin sulfate, and the like can be used.

As hyaluronic acid or a salt thereof, a commercially available aqueous solution of hyaluronic acid or a salt thereof can be used. Specific examples include VISCOAT(R) 0.5 ophthalmic viscoelastic substance containing 3% sodium hyaluronate, 4% chondroitin sulfate sodium, sodium dihydrogen phosphate monohydrate, sodium dihydrogen phosphate anhydrous, and a tonicity agent as components; PROVISC(R) 0.85 ophthalmic viscoelastic substance 1% containing 1% sodium hyaluronate, disodium hydrogen phosphate anhydrous, sodium dihydrogen phosphate monohydrate, pH adjusters (two components), and a tonicity agent as components; OPEGAN(R) 0.6 Ophthalmic Viscoelastic Preparation containing 0.6% sodium hyaluronate, sodium chloride, sodium dihydrogen phosphate, and sodium hydrogen phosphate as components; OPEGAN (R) 1.1 Ophthalmic Viscoelastic Preparation containing 1.1% sodium hyaluronate, sodium chloride, sodium dihydrogen phosphate, and sodium hydrogen phosphate as components; and Hyalein(R) Mini ophthalmic solution 0.3% containing 0.3% sodium hyaluronate, ε-aminocaproic acid, disodium edetate hydrate, potassium chloride, sodium chloride, and a pH-adjuster as components. The commercially available aqueous solution of hyaluronic acid or a salt thereof is preferably free of a substance that affects the viscosity (e.g., a thickener) other than hyaluronic acid and chondroitin sulfate, and is more preferably free of a substance that affects the viscosity (e.g., a thickener) other than hyaluronic acid.

Examples of the "pharmaceutically acceptable aqueous liquid" include an aqueous solution containing a balanced salt (i.e., a balanced salt solution). In the aqueous solution containing a balanced salt, a buffer, a tonicity agent, a pH adjuster, an antioxidant, a chelating agent, or the like can be appropriately selected and contained within a range that does not affect the viability of retinal tissue to be transplanted.

Examples of the buffer include a phosphoric acid buffer, a boric acid buffer, a citrate buffer, a tartaric acid buffer, an acetate buffer, amino acid, and ε-aminocaproic acid.

Examples of the tonicity agent include saccharides such as sorbitol, glucose, and mannitol, polyhydric alcohols such as glycerol and propylene glycol, salts such as sodium chloride, and boric acid.

Examples of the chelating agent include sodium edetate and citric acid.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), and tartaric acid or a salt thereof (sodium tartrate etc.).

Examples of the antioxidant include ascorbic acid, glutathione, sodium hydrogen sulfite, dry sodium sulfite, sodium pyrosulfite, and tocopherol.

Specific examples of the "pharmaceutically acceptable aqueous liquid" include an aqueous solution containing one or multiple of components selected from saccharides such as glucose, inorganic salts such as calcium chloride, sodium chloride, magnesium chloride, potassium chloride, and magnesium sulfate, inorganic materials such as sodium hydrogen carbonate, sodium acetate, sodium citrate, sodium dihydrogen phosphate, sodium hydrogen phosphate, disodium hydrogen phosphate anhydrous, and hydrochloric acid, and chelating agents such as edetate (e.g., sodium edetate).

To prepare the pharmaceutically acceptable aqueous liquid, a commercially available intraocular irrigating/washing solution can be used. Specific examples of intraocular irrigating/washing solution that can be used include an aqueous solution containing 1.5 mg of glucose, 6.6 mg of sodium chloride, 0.36 mg of potassium chloride, 0.18 mg of calcium chloride hydrate, 0.3 mg of magnesium sulfate hydrate, and 2.1 mg of sodium hydrogen carbonate in 1 mL, and sodium citrate hydrate, sodium acetate hydrate, and hydrochloric acid as additives, which is commercially available as OPEGUARD(R)-MA intraocular irrigating solution (Senju Pharmaceutical Co., Ltd.); and an oxiglutatione ocular irrigating solution. In addition, Hank's balanced salt solution (HBSS), Eagle's balanced salt solution (EBSS), phosphate-buffered saline (PBS), Dulbecco's phosphate-buffered saline (DPBS), and the like can also be used to prepare a pharmaceutically acceptable aqueous liquid.

Examples of one aspect of the dispersion vehicle include a vehicle containing a balanced salt solution containing hyaluronic acid and/or chondroitin sulfate as a thickening component and one or more components selected from the above-described buffer, tonicity agent, pH adjuster, antioxidant, chelating agent, and the like as appropriate within a range that does not affect the viability of retinal tissue.

In one aspect of the dispersion vehicle, examples thereof include a vehicle containing only hyaluronic acid as a thickening component, and specifically, ophthalmic viscoelastic substance PROVISC(R) 0.85 ophthalmic viscoelastic substance 1% (Alcon Japan Ltd) containing 10 mg/mL of sodium hyaluronate without chondroitin sulfate can be prepared by 20 to 200-fold dilution (preferably 50 to 150-fold, approximately 100-fold dilution) with a balanced salt solution such as HBSS, for example.

In one aspect of the dispersion vehicle, VISCOAT(R) 0.5 ophthalmic viscoelastic substance (Alcon Japan Ltd) containing 30 mg/mL of sodium hyaluronate and 40 mg/mL of chondroitin sulfate sodium can be prepared by 20 to 200-fold solution (preferably 50 to 150-fold, approximately 100-fold dilution) with a balanced salt solution such as HBSS.

In one aspect of the dispersion vehicle, OPEGAN(R) 0.6 ophthalmic viscoelastic substance can be prepared by 20 to 200-fold dilution with an aqueous liquid such as OPEGUARD. In another aspect, Hyalein(R) Mini ophthalmic solution 0.3% can be prepared by 6 to 60-fold dilution with an aqueous liquid such as OPEGUARD.

In one aspect of the dispersion vehicle, examples thereof include a vehicle containing hyaluronic acid as a thickening component, and may further contain chondroitin sulfate, where the concentration of hyaluronic acid and chondroitin sulfate is not particularly limited, and it is preferable that the viscosity of the dispersion vehicle at a shear rate of 2 (1/s) at 25°C can be maintained at 0.5 to 4 mPa·s, preferably 2 to 3 mPa·s. One aspect of the dispersion vehicle includes a dispersion vehicle containing 0.005 w/v% to 0.15 w/v% (e.g., 0.007 w/v% to 0.04 w/v%, 0.008 w/v% to 0.035 w/v%, 0.009 w/v% to 0.03 w/v%, approximately 0.01 w/v%) of hyaluronic acid with an average molecular weight of 500,000 to 3.9 million (preferably 1.5 million to 3.9 million). Additional examples thereof include a dispersion vehicle containing more than 0 w/v% (e.g., 0.001 w/v%) to 0.20 w/v% (or 0.1 w/v%, 0.05 w/v%) of chondroitin sulfate or a salt thereof with a molecular weight of 20,000 to 24,000. As described above, the dispersion vehicle is more preferably free of chondroitin sulfate.

The dispersion vehicle is preferably free of any thickener (thickening component) other than hyaluronic acid and chondroitin sulfate, or any thickener (thickening component) other than hyaluronic acid. Examples of the thickener include polysaccharides such as gum arabic, gumkaraya, xanthan gum, caseins, agar, alginate, α-cyclodextrin, dextrin, dextran, carrageenan, gelatin, collagen, pectin, starch, chitin and derivatives thereof, chitosan and derivatives thereof, elastin, heparin, heparinoid, heparin sulfate, heparan sulfate, hyaluronic acid, methylcellulose, and hydroxyethyl cellulose or derivatives thereof, ceramide, macrogol, glycerin, povidone, polyvinyl methacrylate, polyvinyl alcohol, polyacrylic acid, carboxyvinyl polymer, and polyethyleneimine.

The dispersion vehicle is preferably free of an antibacterial agent and a preservative.

From the viewpoint of reducing the burden on a patient undergoing administration (transplantation), the dispersion vehicle preferably contains fewer additives as long as the function and stability of retinal pigment epithelial cells are ensured. When a vehicle that is free of thickening components is used, it is expected that the bleb at the time of administration will be re-attached as early as possible, thereby protecting the photoreceptor and maintaining or improving visual function. Therefore, examples of one aspect of the dispersion vehicle include a vehicle containing a balanced salt solution that is free of thickening components and containing one or more components selected from the above-described buffer, tonicity agent, pH adjuster, antioxidant, chelating agent, and the like as appropriate within a range that does not affect the viability of retinal tissue but exhibits physical properties suitable for administration.

In this context, examples of the physical properties suitable for administration include pH and osmotic pressure. The pH of the dispersion vehicle is not particularly limited as long as it is in a neutral range, and the dispersion vehicle may be adjusted to pH 6.5 to 8.0, preferably about pH 7.0 to 7.5. Examples of the osmotic pressure of the dispersion vehicle include hypotonic, isotonic, and hypertonic pressures, and it is preferable to be close to isotonic pressure. The osmotic pressure ratio may be adjusted to 0.7 to 1.3, preferably 0.9 to 1.1.

The dispersion vehicle can be preferably subjected to sterilization treatment such as filter sterilization using a membrane filter.

In other words, when the viscosity of the vehicle used for dispersion of human retinal pigment epithelial cells is within the predetermined range, especially when combined with the administration method described below (air exchange of the vitreous cavity, etc.), the therapeutic agent of the present invention can be said to prevent the formation of the epiretinal membrane or to reduce the degree of the formation of the epiretinal membrane after administration into a human subject. As used herein, the degree of formation of the epiretinal membrane means the degree of formation of the epiretinal membrane as compared with the case where a disease or pathological condition based on a disorder of the retinal pigment epithelium is treated with a therapeutic agent using a dispersion vehicle having a viscosity of more than 4 mPa·s (shear rate of 2 (1/s) at 25°C) (where postoperative air exchange of the vitreous cavity may not be performed) or the case where a disease or pathological condition based on a disorder of the retinal pigment epithelium is treated with a technique of transvitreal subretinal injection using the same dispersion vehicle without performing postoperative air exchange of the vitreous cavity. The expression "the degree of formation of the epiretinal membrane is reduced" may refer to a reduction in the degree of formation of the epiretinal membrane, for example, by 50% or more, 60% or more, 70% or more, 80% or more, or 90% or more as compared with the case where a disease or pathological condition based on a disorder of the retinal pigment epithelium is treated with a therapeutic agent using a dispersion vehicle having a viscosity of more than 4 mPa·s (shear rate 2 (1/s) at 25°C) (where postoperative air exchange of the vitreous cavity may not be performed) or the case where a disease or pathological condition based on a disorder of the retinal pigment epithelium is treated with a technique of transvitreal subretinal injection using the same dispersion vehicle without postoperative air exchange of the vitreous cavity.

The reduction in the degree of formation of the epiretinal membrane can be confirmed based on, for example, the results of fundus examination or optical coherence tomography (OCT) findings.

In other words, when the viscosity of the vehicle used for dispersion of human retinal pigment epithelial cells is within the predetermined range, the therapeutic agent of the present invention can be said to prevent prolonged retinal detachment or to reduce the degree of the prolonged retinal detachment after administration into a human subject. As used herein, the degree of prolonged retinal detachment refers to the degree of prolonged retinal detachment as compared with the case where a disease or pathological condition based on a disorder of the retinal pigment epithelium is treated with a therapeutic agent using a dispersion vehicle having a viscosity of more than 4 mPa·s (shear rate 2 (1/s) at 25°C). The expression "the degree of prolonged retinal detachment is reduced" may refers to a reduction in the degree of prolonged retinal detachment, for example, by 70% or more, 80% or more, 90% or more, or 95% or more as compared with the case where a disease or pathological condition based on a disorder of the retinal pigment epithelium is treated with a therapeutic agent using a dispersion vehicle having a viscosity of more than 4 mPa·s (shear rate 2 (1/s) at 25°C).

The absence of prolonged retinal detachment can be confirmed based on, for example, the results of fundus examination, fundus photography, fluorescein angiography, or optical coherence tomography (OCT) findings.

The human subject to whom the therapeutic agent of the present invention is administered is not particularly limited as long as the human subject has a retinal pigment epithelial tear, and it may be appropriately determined, for example, by the degree and size of the retinal pigment epithelial tear, the degree of visual function, or the like, or by taking these together.

The determination of the administration subject based on the degree and size of retinal pigment epithelial tears may be performed by, for example, the grading system of Sarraf et al. (Retina 30(7), 1039, 45, 2010) or the like. The classification is shown in Table 1 below.

**[Table 1]**

| | |
|---|---|
| Grade 1 | <200 µm |
| Grade 2 | ≥ 200 µm, ≤ 1 disc diameter |
| Grade 3 | > 1 disc diameter |
| Grade 4 | > 1 disc diameter and retinal pigment epithelial tear extending into the fovea |

The human subject to whom the therapeutic agent of the present invention is administered may be a human subject classified in any of grades 1 to 4 above, and is preferably a human subject classified in grades 3 or 4.

It is preferable that the human subject to whom the therapeutic agent of the present invention is administered is a human subject whose visual function is maintained at a certain level or better or whose visual function is likely to be maintained or recovered, although it is affected by a retinal pigment epithelial tear. The maintenance of visual function at a certain level or more may refer to the survival of a certain level or more of photoreceptors in a human subject or may refer to the maintenance of a certain level or more of ocular tissue in a human subject. The visual function can be evaluated by the number of days from onset or diagnosis, findings from ophthalmic examination or the like.

In general, a retinal pigment epithelial tear occurs suddenly, and if the tear occurs in the macula, the patient can be aware of it as a rapid change in vision (decreased visual acuity, scotoma, etc.). If a tear occurs outside the macula, it is difficult to be subjectively recognized, but if the patient regularly visits a medical institution for retinal, choroidal, or other eye-related diseases, it can be easily discovered by funduscopy, OCT, or the like. It is preferable that the human subject to whom the therapeutic agent of the present invention is administered be a human subject in whom the time of onset is specified to some extent, and may be a human subject in whom a long time has not elapsed from the onset. For example, the human subject within 1 year, 10 months, 8 months, 6 months, 4 months, 2 months, 1 month, 2 weeks, or 1 week from the onset or diagnosis is preferable as a subject in whom the visual function is maintained at a certain level or more, or in whom maintenance or recovery of the visual function is possible.

Retinal pigment epithelial tears may occur concomitantly or secondarily in the retina, choroid, or other eye-related diseases, may be caused by trauma, etc., or may be idiopathic retinal pigment epithelial tears with no clear cause.

Examples of the retina-choroid and other eye-related diseases include macular degeneration (e.g., atrophic and exudative age-related macular degeneration), hereditary retinal diseases, central serous chorioretinopathy, choroidal tumors, degeneration of retinal pigment epithelium (including retinitis pigmentosa and crystalline retinopathy), macular dystrophy (including Stargardt disease), cone dystrophy, rod dystrophy, cone-rod dystrophy, high myopia, posterior staphyloma (staphyloma), Harada disease (Vogt-Koyanagi-Harada disease), panuveitis, scleral surgery, and complications in retinal detachment surgery and glaucoma surgery, and the like. Therefore, the therapeutic agents for retinal pigment epithelial tears in the present specification include therapeutic agents for retinal pigment epithelial tears that are transplanted in patients diagnosed with any of the above diseases (e.g., macular degeneration).

Examples of the ophthalmic examination include visual acuity examination (examination using a Landolt ring, an ETDRS visual acuity chart, a pictorial visual target, a stripe visual target, or the like, visual behavior, etc.), color vision examination, contrast examination, OCT examination, dynamic visual field examination, static visual field examination, fundus photography, fluorescent fundus angiography, fundus autofluorescence photography, electroretinography (ERG) examination, full-field stimulus testing (FST), and visual evoked potential (VEP) examination.

The human subject whose visual function is maintained at a certain level or higher may be, for example, a subject whose best corrected visual acuity (BCVA) using the ETDRS visual acuity chart is, in terms of letter score, 0 letters or more and 85 letters or less (corresponding to a decimal visual acuity of 0.02 to 1.0), or 24 letters or more and 78 letters or less. A subject with a letter score of 100 letters or less (corresponding to a decimal visual acuity of 2.0) may also be included as a human subject if there is a visual field defect or the like.

The human subject whose visual function is maintained at a certain level or more may be, for example, a human subject having a central visual field angle of 28 degrees or more with a Goldmann-type perimeter I/2 visual target.

The human subject whose condition of ocular tissues is maintained or likely to be maintained at a certain level or more may be, for example, a human subject having no damage other than the retinal pigment epithelial cell layer, a human subject in which at least the external limiting membrane (ELM) and the inner segment/outer segment junction (IS/OS-line) are maintained, or a human subject in whom the outer nuclear layer(ONL) of the retinal pigment epithelial tear site is not thinned as compared with the surrounding normal site.

The HLA has mainly six loci antigens, A, B, C, DR, DQ, and DP, each of which is composed of complex combinations of dozens of different types (alleles), and there are tens of thousands of such combinations. In general organ transplantation, particularly bone marrow transplantation, it is considered necessary, in principle, to match all six loci of the major HLA antigens, A, B, C, DR, DQ, and DP.

The human subject to whom the therapeutic agent of the present invention is administered may or may not have a human leukocyte antigen (HLA) type that matches that of the human retinal pigment epithelial cells, but it is preferable that they do match. If the human subject is HLA type matched with retinal pigment epithelial cells in the present invention, all HLA antigens of HLA-A, -B, -C, -DR, -DQ and - DP of the human subject and retinal pigment epithelial cells are the same. When a human subject is HLA-mismatched with a retinal pigment epithelial cell in the present invention, at least one HLA antigen of HLA-A, -B, -C, -DR, -DQ and -DP may be different between the HLA type of the human subject and the HLA type of the retinal pigment epithelial cell, or all of them may be different. In the present specification, a human subject may be included in the case where the human subject is HLA-matched with a retinal pigment epithelial cell in the present invention when the genotypes at the HLA-A locus, the HLA-B locus and the HLA-DR locus of the retinal pigment epithelial cell are each homozygous, and when the genotype of at least one allele at the locus of the human subject corresponding to the locus of the retinal pigment epithelial cell matches the genotype of the allele at the locus of the retinal pigment epithelial cell.

The retinal pigment epithelial tear in a human subject is preferably in a state where a retinal pigment epithelial cell layer of the human subject is partially ruptured, and Bruch's membrane exposed without being covered with retinal pigment epithelial cells of the human subject remains as a basement membrane under the retina. The Bruch's membrane is a thin membrane present between the retinal pigment epithelial cell layer and the choroid, and is composed of type IV collagen, laminin, and the like. The Bruch's membrane remaining as a basement membrane in the subretinal space of the ocular tissue means that there is no loss or defect of the Bruch's membrane in the subretinal space of the ocular tissues, and there is a scaffold to which the administered retinal pigment epithelial cells adhere. It is preferable that the remaining Bruch's membrane be not damaged, but it may be damaged as long as no loss or defect occurs. The Bruch's membrane is composed of five layers in order from the retinal side: the basal lamina of retinal pigment epithelial cells, the inner collagenous fiber layer, the elastic fiber layer, the outer collagenous fiber layer, and the basal lamina of choroidal capillary endothelial cells. In one aspect, the loss or defect of the Bruch's membrane refers to a case where the surface layer (the basal lamina of retinal pigment epithelial cells) on the retinal side is especially lost or deficient.

As shown in Examples, human retinal pigment epithelial cells adhere well when seeded on a substrate coated with properties similar to those of the components constituting the Bruch's membrane, but cell adhesion is reduced when the coating substrate is absent or detached. Therefore, the therapeutic agent of the present invention would be highly effective in treating under the condition in which the Bruch's membrane remains as a basement membrane under the retina. After administration of the therapeutic agent of the present invention to the human subject, the human retinal pigment epithelial cells become polarized and form an epithelial layer to cover the exposed Bruch's membrane, and exert a barrier function. The expression "become polarized" means that the cells display characteristic protein localization patterns, with a functional bias at the apical and basolateral membranes. In addition, the expression "exert a barrier function" means that the retinal pigment epithelial cells adhere tightly to each other by tight junctions, strictly regulating the movement of materials and maintaining the intraocular environment necessary for neuronal activity.

The phrase "retinal pigment epithelial tear is in a state where a retinal pigment epithelial cell layer of the human subject is partially ruptured, and Bruch's membrane exposed without being covered with retinal pigment epithelial cells of the human subject remains as a basement membrane under the retina" may refer to, for example, a retinal pigment epithelial tear of a human subject with a grade of 3 to 4, a retinal pigment epithelial tear of a human subject with a number of days from onset or diagnosis within the above range, a neural retina (photoreceptors) of a human subject not in contact with the retinal pigment epithelial cell layer, a rupture or disappearance of the Bruch's membrane not clearly confirmed by imaging examination such as OCT, and the like.

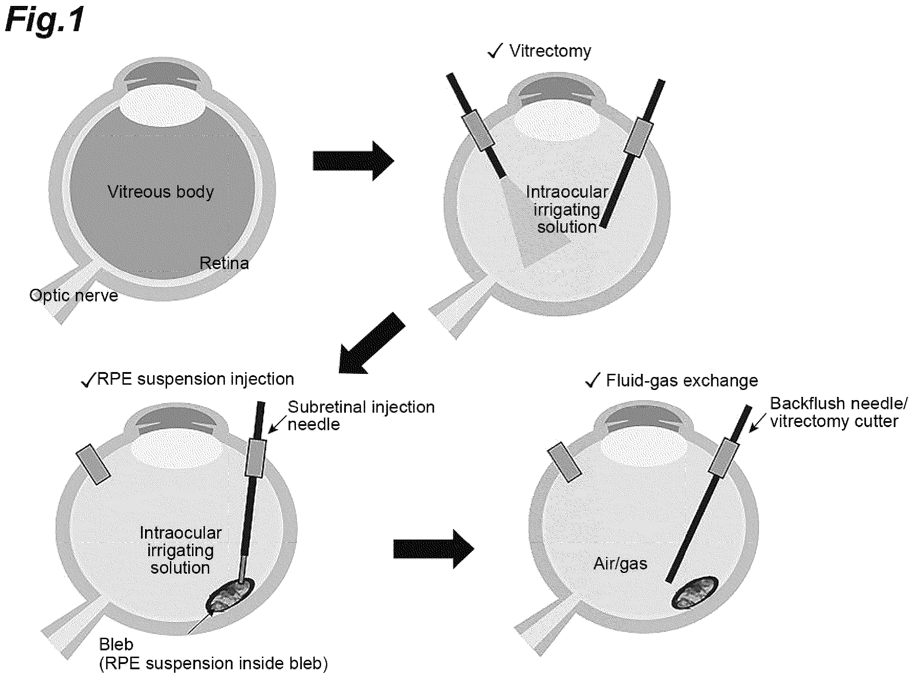

The subretinal administration (transplantation) method of the therapeutic agent of the present invention is not particularly limited as long as it is a known method, and examples thereof include injection using a syringe of an appropriate size or a transplantation device comprising a needle or a resin part. More specifically, the examples include the transvitreal method and the transchoroidal approach. As outlined in Figure 1, the transvitreal method may be performed by using a cataract and vitreous surgical equipment to conduct a vitrectomy including the posterior vitreous detachment, and then administering an appropriate amount of the therapeutic agent of the present invention by injection into the subretinal space (between the sensory retina and retinal pigment epithelial cells) to form a bleb (hydraulically debrided retinal detachment). After administration, the vitreous cavity may be replaced with air if necessary. Besides the method of forming a bleb through injection of the therapeutic agent of the present invention itself, the method of forming a bleb includes a method of injecting the therapeutic agent of the present invention after first injecting an ocular irrigating solution (such as HBSS) to form a bleb and, if necessary, aspirating the liquid in the bleb.

It is preferable that the administration method of the therapeutic agent of the present invention be the transvitreal method, and it is preferable to replace the vitreous with air after the subretinal transplantation by the transvitreal method.

In the transvitreal method, a few ports are made on the sclera for the insertion of surgical instruments. Since the therapeutic agent of the present invention can be administered (injected) using a needle, a syringe, or the like, the method can be performed in a minimally invasive manner among transvitreal methods. When the therapeutic agent of the present invention is administered by the transvitreal method, the therapeutic agent of the present invention may be administered through the ports of about 0.4 mm to about 0.65 mm on the sclera to the region surrounding the retinal pigment epithelial tear while forming a bleb. The choroidal damage and hemorrhage can also be avoided by treating the region surrounding the retinal pigment epithelial tear. The port on the sclera may be a port of about 0.4 mm (27 gauge) to about 0.63 mm (23 gauge) (e.g., about 0.5 mm (25 gauge)) depending on the size of the needle. It is preferable that the bleb be formed to cover a major axis of the retinal pigment epithelial tear. The region surrounding the retinal pigment epithelial tear is more preferable as the distance from the edge of the retinal pigment epithelial tear is shorter, and may be, for example, a region within one disc diameter (about 1.5 mm) around the edge of the retinal pigment epithelial tear.

When the therapeutic agent of the present invention is administered through the ports of about 0.4 mm to about 0.65 mm on the sclera to the region surrounding the retinal pigment epithelial tear while forming a bleb, it is preferable to use the therapeutic agent for treatment including air exchange of the vitreous after administration.

The transchoroidal approach is a method of approaching from the outside of the eyeball, in which a portion of the sclera is incised and detached, a catheter is inserted into the choroidal space therefrom, and an appropriate amount of the therapeutic agent of the present invention can be administered by subretinal injection.

The injection needle is not particularly limited, and examples thereof includes a metallic bevel needle and a polytip needle. A polytip needle is particularly preferred. In one aspect, the outer diameter of the injection needle may be 38 gauge, 39 gauge, or 40 gauge.

In the transvitreal method, the transplantation composition or the retinal pigment epithelial cells for transplantation may leak from the bleb into the vitreous, and the leaked retinal pigment epithelial cells may form the epiretinal membrane. On the other hand, in the transchoroidal approach, the transplantation composition rarely leaks into the vitreous unless a hole is made on the retinal side. However, the epiretinal membrane may be formed by retinal pigment epithelial cells leaking from the bleb, even in the transchoroidal approach, because the needle may perforate the retinal side upon administration of a liquid into a narrow space. In addition, both the transvitreal method and the transchoroidal approach cause retinal detachment. The formation of the epiretinal membrane and the prolonged retinal detachment are considered as adverse effects of subretinal administration. As described above, the dispersion vehicle in the therapeutic agent of the present invention can suppress cell leakage of the therapeutic agent of the present invention into the vitreous and prolonged retinal detachment by having a specific viscosity.

The therapeutic agent of the present invention may be administered in combination with an immunosuppressive agent. The immunosuppressive agent to be administered in combination may be a single type or include multiple types. Examples of the immunosuppressive agent include a steroidal anti-inflammatory drug, a calcineurin inhibitor, an antimetabolite, and a mTOR inhibitor. Examples of the steroidal anti-inflammatory drug include dexamethasone and prednisolone, examples of the calcineurin inhibitor include cyclosporine and tacrolimus, examples of the antimetabolite include mycophenolic acid, and examples of the mTOR inhibitor include sirolimus (rapamycin). In addition, betamethasone sodium phosphate and/or fluorometholone may be combined. The immunosuppressive agent is used for the purpose of preventing rejection of the therapeutic agent of the present invention (retinal pigment epithelial cells). The steroidal anti-inflammatory drugs, dexamethasone, betamethasone sodium phosphate and/or fluorometholone may be used for the purpose of inflammatory suppression after surgery.

In one aspect, triamcinolone acetonide may be administered topically (e.g., subtenon, intravitreal), and cyclosporine, tacrolimus, dexamethasone, and prednisolone may be administered systemically (e.g., orally, intravenously, etc.). Dexamethasone may be administered subconjunctivally, and betamethasone sodium phosphate and fluorometholone may be instilled.

In one aspect, prednisolone and cyclosporine or tacrolimus are administered before surgery. Administration may start up to 7 days (6, 5, 4, 3, 2, or 1 day) before surgery. Triamcinolone acetonide, dexamethasone, prednisolone, and cyclosporine or tacrolimus may be administered after surgery. Betamethasone sodium phosphate and fluorometholone may be administered after surgery.

The therapeutic agent of the present invention may be prepared in a suspension using the above-described dispersion vehicle or the like in a medical institution. In this case, the retinal pigment epithelial cells may be frozen and transported to the medical institution as a cryopreserved formulation containing the retinal pigment epithelial cells. It is preferable that the retinal pigment epithelial cells be frozen in a dispersed state. The method of cryopreservation of retinal pigment epithelial cells is not limited as long as it is a method generally known for cryopreserving cells. For example, the cells can be frozen at -80°C and then cryopreserved with liquid nitrogen by using a commercially available cell cryopreservation solution StemCellBanker (Zenoaq). In a medical institution, the cryopreserved formulation may be thawed at the time of use and suspended in a dispersion vehicle or the like for use in transplantation medicine. After thawing of the cryopreserved formulation, the cells may be washed using a buffer solution such as a dispersion vehicle before suspension in the dispersion vehicle or the like.

Therefore, one aspect of the present invention also provides a cryopreserved formulation containing retinal pigment epithelial cells, for use as the therapeutic agent of the present invention by preparing a suspension using the above-described dispersion vehicle or the like after thawing. In other words, the present invention provides a cryopreserved formulation containing retinal pigment epithelial cells, for use in the treatment of a retinal pigment epithelial tear in a human subject as a suspension of human retinal pigment epithelial cells dispersed after thawing.

### 3. Method of Treatment

The method of treatment for a retinal pigment epithelial tear in a human subject of the present invention includes administering to a human subject in need thereof an effective amount of a suspension of dispersed human retinal pigment epithelial cells. As the suspension of dispersed human retinal pigment epithelial cells, the therapeutic agent described in 2. Therapeutic Agent above may be used.

As described above, the method of administering the suspension of dispersed human retinal pigment epithelial cells is not particularly limited as long as it is a known method, and examples thereof include the transvitreal method and the transchoroidal approach. It is preferable that in the method of treatment of the present invention, the administration of the suspension of dispersed human retinal pigment epithelial cells include administration by the transvitreal method.

Human subjects receive local (sub-Tenon's anaesthesia/ retrobulbar anaesthesia) or general anesthesia. When the suspension is to be administered by the transvitreal method, vitrectomy is done using an appropriate vitreous surgical equipment, (e.g., CONSTELLATION Vision System). At this time, if necessary, triamcinolone acetonide (0.5 to 4 mg) may be injected into the vitreous for the purpose of vitreous visualization. The suspension of dispersed human retinal pigment epithelial cells may be administered into the region surrounding the retinal pigment epithelial tear through a port of about 0.4 mm to about 0.65 mm on the sclera to form a bleb. As a result, the retinal pigment epithelial tear in the human subject can be treated in a minimally invasive manner. The port on the sclera may be a port of about 0.4 mm (27 gauge) to about 0.63 mm (23 gauge) (e.g., about 0.5 mm (25 gauge)) depending on the size of the needle. It is preferable that the bleb be formed to cover a major axis of the retinal pigment epithelial tear.

The method of treatment of the present invention may further include washing the vitreous cavity and air-exchanging the vitreous cavity of the human subject after administration of the suspension, where the administration of the suspension of dispersed human retinal pigment epithelial cells involves blebbing through ports of about 0.4 mm to about 0.65 mm on the sclera in the region surrounding the retinal pigment epithelial tear.

In the method of treatment of the present invention, the immunosuppressive agent described above may be administered in combination.

It is preferable to rest in a supine position for at least 12 hours after implementation of the method of treatment of the present invention.

In the method of treatment of the present invention, the aspect of the therapeutic agent of the present invention as described above is similarly applicable.

As an effect of administration (transplantation) of the therapeutic agent or implementation of the method of treatment of the present invention, retinal pigment epithelial cells are engrafted at the administration site of the human subject, whereby the adhesion structure with the photoreceptor layer is reconstructed. As a result, the transplanted retinal pigment epithelial cells function to restore visual function or prevent further deterioration and maintain visual function as compared with conditions prior to administration of the therapeutic agent or implementation of the method of treatment.

The above therapeutic effect can be confirmed by the above-described ophthalmologic examinations. It can also be assessed with scales that measure health-related quality of life (QOL). These scales comprehensively assess the health condition from physical, mental, and social aspects, and there are also indices dedicated to visual function. Examples thereof include the Visual Function Questionnaire (VFQ-25), the Euro-QOL 5 Dimension-5 Level (EQ-5D-5L), the Health Utilities Index Mark 3 (HUI3).

### 4. Use

The present invention provides use of a suspension of dispersed human retinal pigment epithelial cells in the treatment of a retinal pigment epithelial tear in a human subject. The present invention also provides a suspension of dispersed human retinal pigment epithelial cells, for use in treatment of a retinal pigment epithelial tear in a human subject. The present invention further provides use of a suspension of dispersed human retinal pigment epithelial cells in production of a therapeutic agent for a retinal pigment epithelial tear in a human subject.

### Examples

### <Example 1: Study of Influence on Cell Leakage Using Enucleated Porcine Eye>

The influence of the viscosity of the dispersion vehicle and postoperative air exchange in the vitreous cavity on cell leakage was examined *ex-vivo* using porcine eyes having an eyeball size close to that of a human. Retinal pigment epithelial (RPE) cells derived from human iPS cells (QHJI01s04 line) produced in the same manner as the method of Kuroda et al. (Stem Cell Res. 2019; 39:101514.) were used in all Examples. Porcine eyes extracted from pigs for meat production were obtained on the same day, and when the fundus was difficult to observe due to lens opacity, the lens was removed if necessary. A suspension of human iPS cell-derived RPE cells were subretinally administered (transplanted) by the method described below.

As shown in the schematic view of Figure 1, subretinal administration of the RPE cell suspension was conducted according to a general procedure of vitreous surgery (transvitreal method). Using a cataract and vitreous surgical equipment (CONSTELLATION Vision System LTX, manufactured by Alcon Japan Ltd), 0.5 to 4 mg of triamcinolone acetonide (manufactured by FUJIFILM Wako Pure Chemical Corporation, 209-10961) was injected intravitreally to visualize the vitreous, and posterior vitreous detachment was created from the macula and peripheral vitrectomy was performed. An ophthalmic surgical syringe and needle adapter was connected to the vitreous surgical equipment using an appropriate tube and filled with RPE cell suspension. A subretinal injection needle (MedOne Cannula: Tapered PolyTip Cannula 25G/38G, 3219, MedOne Surgical, Inc./NIHON SURGE Inc.) was connected after removal of air at the tip using the ejection function of the vitreous surgical equipment. The subretinal injection needle was inserted through the port, and the RPE cell suspension was subretinally administered by injection in an appropriate volume. Subretinal administration of the RPE cell suspension resulted in bleb formation. After administration, the vitreous cavity was thoroughly washed and, if necessary, exchanged with air.

The administered RPE cell suspension was obtained by suspending RPE cells labeled with the fluorescence labeling reagent PKH26 red fluorescent cell linker Mini kit (manufactured by Sigma-Aldrich, MINI26) in two types of transplant media at a concentration of 5.7 × 10⁷ cells/mL (air exchange condition) or 6.0 × 10⁶ cells/mL (no air exchange).
Viscous vehicle: Vehicle obtained by mixing VISCOAT 0.5 ophthalmic viscoelastic substance (manufactured by Alcon Japan Ltd) and Hank's balanced salt solution HBSS(+)(manufactured by Thermo Fisher Scientific, Inc., 14025092) at a ratio of 1:6 (7-fold dilution).
Buffer only vehicle: HBSS(+) only vehicle

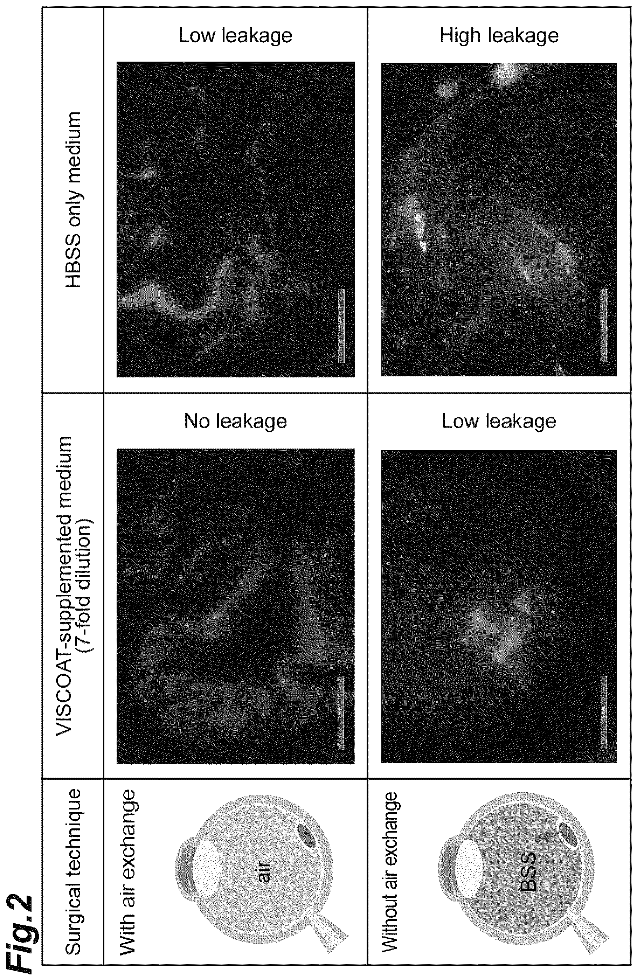

After administration, the porcine eye was allowed to stand for several hours, and the hemisphere on the corneal side was removed to perform fundus fluorescence observation under a stereo fluorescence microscope (Leica M165FC, manufactured by Leica Microsystems), and the results are shown in Figure 2. It was found from Figure 2 that cell leakage was reduced by adding hyaluronic acid to the vehicle. In addition, no cell leakage was observed with the combined use of vitreous air exchange.

In addition, subretinal administration was carried out by varying the dilution factor of VISCOAT in the vehicle in the range of 7 to 21 times. It was found that the higher concentration of VISCOAT (lower dilution factor) resulted in higher resistance during administration, making it difficult to form the bleb. It was also confirmed that a dose volume of approximately 100 µL could be administered, but reflux from the injection point was observed when the dose volume was increased. It was confirmed that the RPE cells up to a concentration of 5.7 × 10⁷ cells/mL could be successfully administered without any problem.

### <Example 2: Transplantation Experiment in Cynomolgus Monkey 1>

To reduce cell leakage and improve subretinal cell engraftment, a administration method was examined using cynomolgus monkeys, which have the macula and ocular anatomy very close to that of humans. Human iPS cell-derived RPE cells suspended in HBSS (+) (same as above) only or in a dispersion vehicle prepared by diluting the ophthalmic viscoelastic substance such as PROVISC(R) 0.85 ophthalmic viscoelastic substance 1% (Alcon Japan Ltd) (hereinafter referred to as PROVISC) or VISCOAT(R) 0.5 ophthalmic viscoelastic substance (Alcon Japan Ltd) (hereinafter referred to as VISCOAT) by 20-, 100-, or 200-fold dilution with HBSS (+) were subretinally administered to cynomolgus monkeys to examine the appropriate composition of the dispersion vehicle. In addition, the effect of the presence or absence of postoperative air exchange of the vitreous cavity was also examined. Herein, VISCOAT contains 30 mg/mL of sodium hyaluronate and 40 mg/mL of chondroitin sulfate sodium, and PROVISC contains 10 mg/mL of sodium hyaluronate without chondroitin sulfate.

Cambodian native cynomolgus monkeys (2.0 to 5.0 kg at the onset of habituation) were used in the study. One to two drops of tropicamide/phenylephrine hydrochloride ophthalmic solution (Mydrin(R)-P ophthalmic solution, Santen Pharmaceutical Co., Ltd.) and phenylephrine hydrochloride ophthalmic solution (Neosynesin Kowa 5% ophthalmic solution, Kowa Company, Ltd.) were each applied to the right eyes, to dilate the pupils. 10 mg/kg of Ketamine Hydrochloride (Ketalar for intramuscular injection 500 mg, Daiichi Sankyo Propharma Co., Ltd.) and 0.08 mg/kg of medetomidine hydrochloride (Domitor(R), Orion Corporation) were administered intramuscularly, followed by endotracheal intubation and controlled breathing using a mixed gas of oxygen and 0.5% to 2.0% isoflurane (ISOFLURANE Inhalation Solution "Pfizer", Mylan Inc.). The animals were fixed on the operating table in the supine position, and an appropriate amount (50 to 100 µL) of RPE cell suspension (2.5 × 10⁶ to 1.5 × 10⁷ cells/mL) was subretinally administered in the same manner as in Example 1. After administration, any leaked suspension was aspirated. In addition, the vitreous cavity was replaced with air as in Example 1, if necessary. The supine position was maintained under inhalation anesthesia for up to 3 hours to promote cell sedimentation and engraftment. To suppress rejection, immunosuppression was conducted using a once-daily intramuscular injection of cyclosporine (Sandimmun(R) for i.v. infusion 250 mg, Novartis Pharma K.K.) and sub-Tenon's injection of triamcinolone acetonide (MaQaid(R) OPHTHALMIC INJECTION 40 mg, WAKAMOTO PHARMACEUTICAL CO., LTD.) at surgery. After administration, the administered eyes were observed over time using a fundus camera (Kowa Optimized, VX-10α) and an OCT device (Heidelberg Engineering, SPECTRALIS OCT2).

The results of subretinal administration in cynomolgus monkeys are shown. When a suspension at a cell concentration of 1.5 × 10⁷ cells/mL was used, visibility of the injection needle tip was reduced at the time of injection in all media, while injection was possible without any problem at a cell concentration of 1.0 × 10⁷ cells/mL or less. Regarding the administration volume, it was confirmed that subretinal administration to one eye was possible in a range of 50 to 100 µL, and stable administration was possible with good reproducibility up to a dose volume of 70 to 80 µL.

The results of OCT findings up to 4 weeks later are shown in Table 2 and Table 3. From Table 2 and Table 3, the formation of epiretinal membrane was observed in 7 out of 8 animals in which the vitreous cavity was not replaced with air and in 8 out of 23 animals in which the vitreous cavity was replaced with air, indicating the possibility that the leakage of the cell suspension could be suppressed and the formation of epiretinal membrane could be reduced by fluid air exchange.

In the groups of dispersion media with 20-fold dilution of PROVISC or VISCOAT, prolonged retinal detachment around the administration site was observed in 8 out of 10 cases, while in the other groups, re-attachment of the retina at the administration site was confirmed by approximately 4 weeks after administration, except for one case of 200-fold diluted PROVISC. The vehicle with 20-fold dilution of PROVISC or VISCOAT did not have the suppressing effect on the epiretinal membrane formation. High viscosity reduced the leakage of suspensions, while prolonged retinal detachment left subretinal fluid for a long period of time, which might not have affected the sum of cells leaking to the vitreous side. In addition, the epiretinal membrane was observed more frequently in the dispersion vehicle with 200-fold dilution of PROVISC or VISCOAT. Although the reason is not clear, it is considered that leakage occurs at a viscosity equal to or less than a certain threshold value, and when the leaked hyaluronic acid is present in the vitreous cavity, cells are likely to form the ERM using the hyaluronic acid as a scaffold. On the other hand, the formation of the epiretinal membrane was not observed when a dispersion vehicle with 100-fold dilution of PROVISC or VISCOAT was used. Therefore, it was found that addition of 100-fold diluted PROVISC or VISCOAT to the vehicle is preferred for inhibition of cell leakage.

**[Table 2]**

| Table 2. Prolonged Retinal Detachment/Formation of Epiretinal Membrane | | | |
|---|---|---|---|
| PROVISC/VISCOAT | Prolonged retinal detachment | Epiretinal membrane without air exchange | Epiretinal membrane with air exchange |
| 20-fold dilution | 8/10 | 4/5 | 2/5 |
| 100-fold dilution | 0/3 | - | 0/3 |
| 200-fold dilution | 1/4 | - | 3/4 |
| No addition | 0/14 | 3/3 | 3/11 |

**[Table 3]**

| Table 3. Prolonged Retinal Detachment/Formation of Epiretinal Membrane | | | | |
|---|---|---|---|---|
| Dilution factor | Ophthalmic viscoelastic substance | Prolonged retinal detachment | Epiretinal membrane without air exchange | Epiretinal membrane with air exchange |
| 20-fold dilution | PROVISC | 3/4 | 1/2 | 1/2 |
| | VISCOAT | 5/6 | 3/3 | 1/3 |
| 100-fold dilution | PROVISC | 0/2 | - | 0/2 |
| | VISCOAT | 0/1 | - | 0/1 |
| 200-fold dilution | PROVISC | 0/2 | - | 1/2 |
| | VISCOAT | 1/2 | - | 2/2 |
| - | HBSS(+) only | 0/14 | 3/3 | 3/11 |

At the end of the observation period (4, 13, and 26 weeks after surgery), the eyeballs were enucleated, fixed, and embedded in paraffin. Ten sections (3 to 4 µm thick) were prepared at 0.56 mm intervals to cover the blebs, and in order to evaluate engraftment, fluorescent immunostaining was performed according to a conventional method using one of the RPE cell markers, anti-human PMEL17 antibody (Novus Biologicals, NBP2-47780AF647, mouse or Abcam, ab137062, rabbit), and anti-human CD147 antibody (R&D Systems, AF972, goat) as human cells. The stained specimens were observed with a fluorescence microscope (KEYENCE, BZ-X810) or a slide scanner (Zeiss, Axio Scan. Z1), and the regions including the positive sites were then imaged.

Although the anti-CD147 antibody used showed a strong positive signal in a part of the region where human iPS cell-derived RPE cells were supposed to be engrafted, it was suggested that the anti-CD147 antibody also cross-reacted with monkey antigens since the entire retinal pigment epithelium layer of cynomolgus monkey was stained. Therefore, the anti-PMEL17 antibody-positive signal was used as a major indicator of engraftment and distribution of human iPS cell-derived RPE cells, and the anti-CD147 antibody was used in multiple staining to facilitate the identification of the monkey retinal pigment epithelial layer during observation.

To quantitatively analyze the engraftment performance, the total score (0 to 30) in each individual was calculated by summing the results of 10 slices evaluated on the following four-level grading (Grades 0 to 3) for each slice according to the total number of engraftment sites with consecutive anti-PMEL17 antibody positivity (sites with consecutive subretinal positive cells observed over 100 µm or longer).

### Four-Level Grading

Grade 0: no positive cells
Grade 1: minimal (no consecutive engraftment but positive cells were observed)
Grade 2: moderate (one or two consecutive engraftment sites were observed)
Grade 3: significant (three or more consecutive engraftment sites were observed)

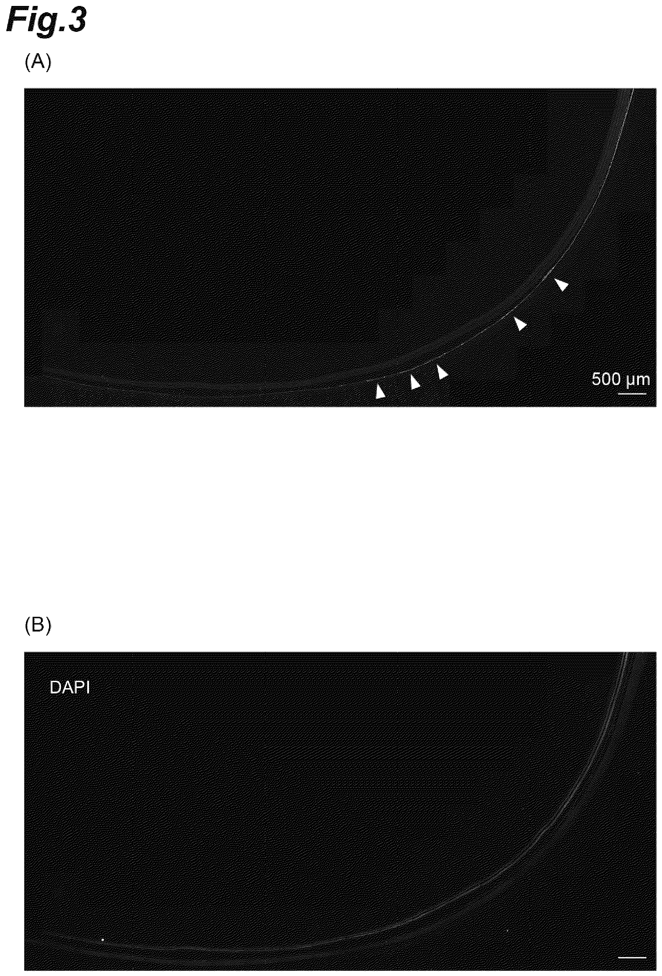

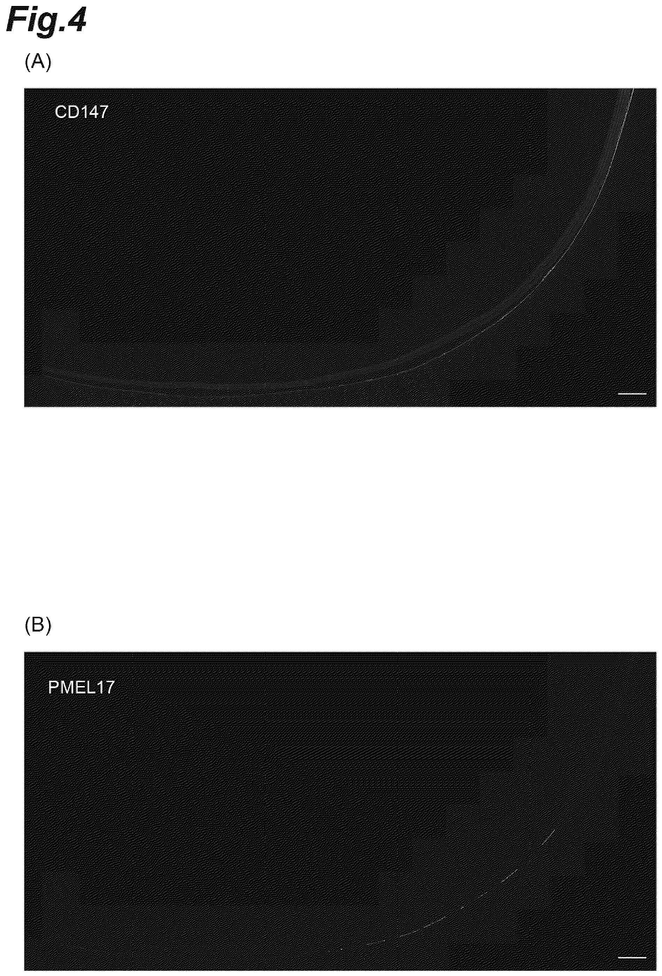

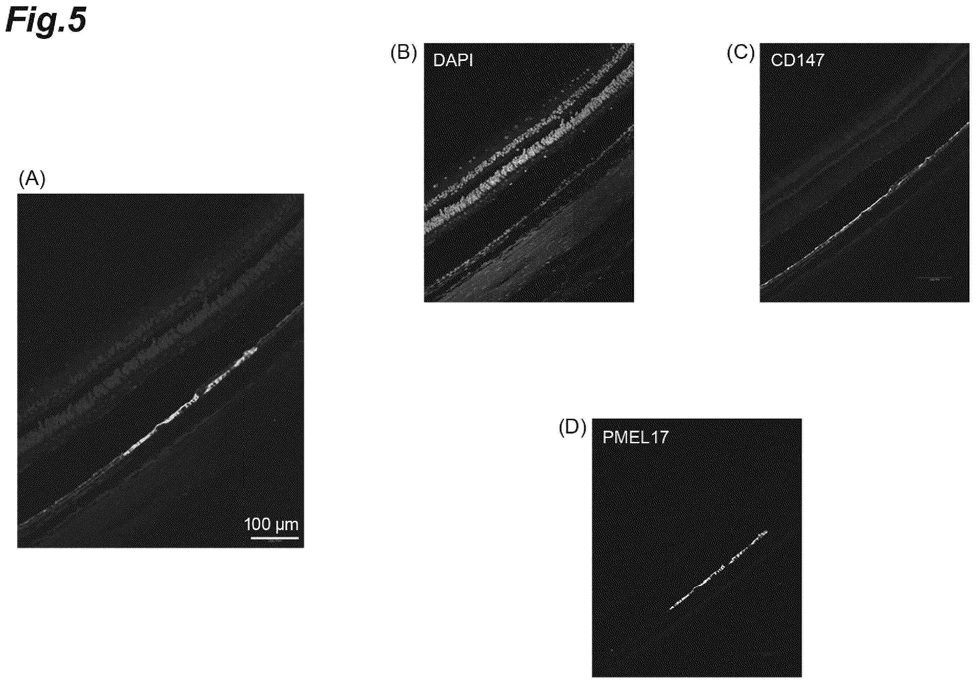

PMEL17 positive sites were observed in most of the individuals. Many of the positive sites were observed in a monolayer in the monkey retinal pigment epithelial cell layer, confirming that the administered human iPS cell-derived RPE cells were integrated into and engrafted into the monkey retinal pigment epithelial layer. Representative low-magnification fluorescence immunostaining images of the 100-fold diluted PROVISC group (Individual No. 1, 4 weeks after surgery) are shown in Figures 3 and 4. Figure 3 (A) is an image of each stain superimposed, Figure 3 (B) shows an image of DAPI staining only, Figure 4 (A) shows an image of CD147 staining only, and Figure 4 (B) shows an image of PMEL17 staining only. The sites indicated by the arrows in Figure 3(A) represent sites of consecutive human iPS cell-derived RPE cell engraftment, and five sites of consecutive engraftment in this individual (Individual No. 1) were identified as Grade 3. Figure 5 shows high-magnification fluorescent immunostaining images (a partial region in Figures 3 and 4) of Individual No. 1 (4 weeks after surgery), where Figure 5(A) is an image of each stain superimposed, and Figure 5(B) to (D) are images of DAPI, CD147, and PMEL17 single staining, respectively.

Engraftment was scored for the hyaluronic acid-supplemented vehicle (100-fold dilution) groups, which showed good inhibition of cell leakage. In addition, the score per unit cell number was calculated because the dose volume (total number of cells administered) varied from individual to individual. The results are shown in Table 3. It was confirmed from Table 4 that the PROVISC condition resulted in wide-range engraftment of the administered cells, whereas the VISCOAT condition resulted in engraftment at only a few sites.

**[Table 4]**

| Individual No. | Transplantation vehicle | Suspension concentration (cell/mL) | Dose volume (µL) | Observation period (week) | Number of slices with grade ≥ 1 | Total score | Score/ 10⁵ cells |
|---|---|---|---|---|---|---|---|
| 1 | 100-fold diluted PROVISC | 5×10^6 | 60 | 4 | 8 | 20 | 6.67 |
| 2 | | 5×10^6 | 80 | 4 | 10 | 21 | 5.25 |
| 3 | 100-fold diluted | 5×10^6 | 50 | 4 | 2 | 3 | 1.20 |
| 4 | VISCOAT | 5×10^6 | 100 | 4 | 2 | 3 | 0.60 |

Based on the above, it was found that the dispersion vehicle containing sodium hyaluronate with an average molecular weight of 1.5 to 3.9 million at a specific concentration is the most suitable for preventing the leakage of the administered cells into the vitreous cavity and achieving wide-range engraftment.

### <Example 3: Transplantation Experiment 2 in Cynomolgus Monkey>

In order to eliminate the influence of immune rejection in a human-monkey xenograft system as much as possible, the influence of the transplantation vehicle on the engraftment of RPE cells was evaluated by enhancing the immunosuppression of cynomolgus monkeys. The suspension of human iPS cell-derived RPE cells was administered in the same manner as in Example 2 into the subretinal space of the right eyes of cynomolgus monkeys immunosuppressed by using the protocol of Example 2 in combination with oral administration of mycophenolate mofetil (CELLCEPT suspension powder 31.8%, Chugai Pharmaceutical Co., Ltd.) once a day and further administering twice the dose of cyclosporine. As a subretinal injection needle, a MicroTip Beveled Cannula (25 g/40 g) (#3261 manufactured by MedOne Surgical, Inc.) was used. To compare the transplantation media under the same test conditions, 50 µL of a cell suspension (1.0 × 10⁷ cells/mL) prepared using HBSS(+) or PROVISC diluted 100-fold with HBSS(+) was injected into the upper and lower parts of the same eye, respectively. In all cases, the vitreous cavity was replaced with air after the surgery, ophthalmologic examination was performed over time, and then the transplanted eyes were histologically analyzed by fluorescent immunostaining. In the histological analysis, the eyeballs were enucleated and fixed after the end of the 4-week observation period, and paraffin embedding was performed. Serial sections (3 to 4 µm thick) were prepared at about 0.28 mm intervals to cover the entire blebs, and fluorescent immunostaining was performed according to a conventional method using anti-human PMEL17 antibody (Novus Biologicals, NBP2-47780AF647, mouse) and anti-human CD147 antibody (R&D Systems, AF972, goat). A slide scanner (Zeiss, Axio Scan. Z1) was used to image the region including the human PMEL17-positive site of the stained specimen to perform the engraftment evaluation of human iPS cell-derived RPE cells.

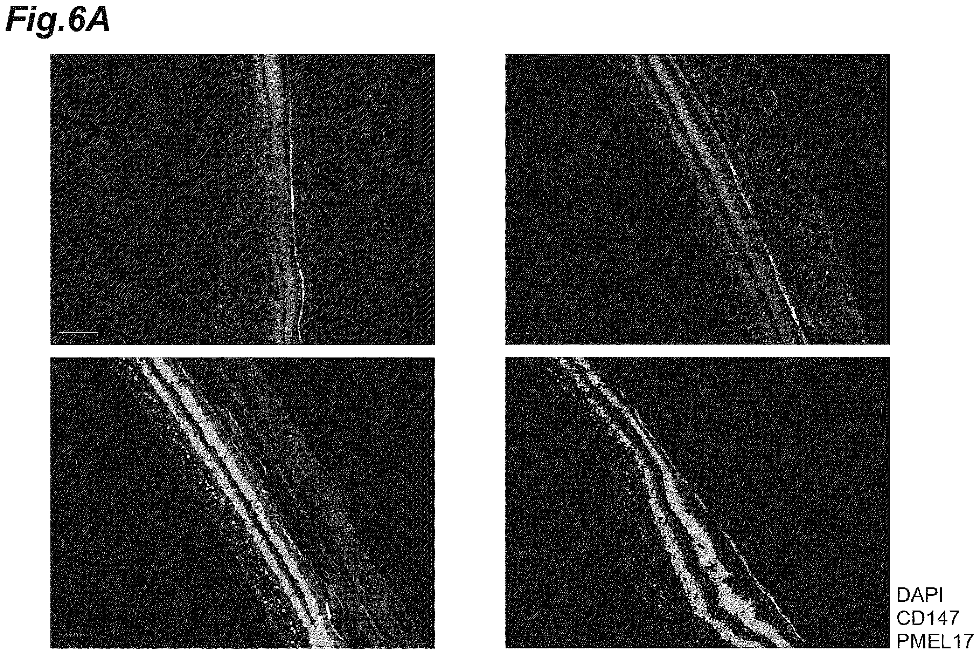

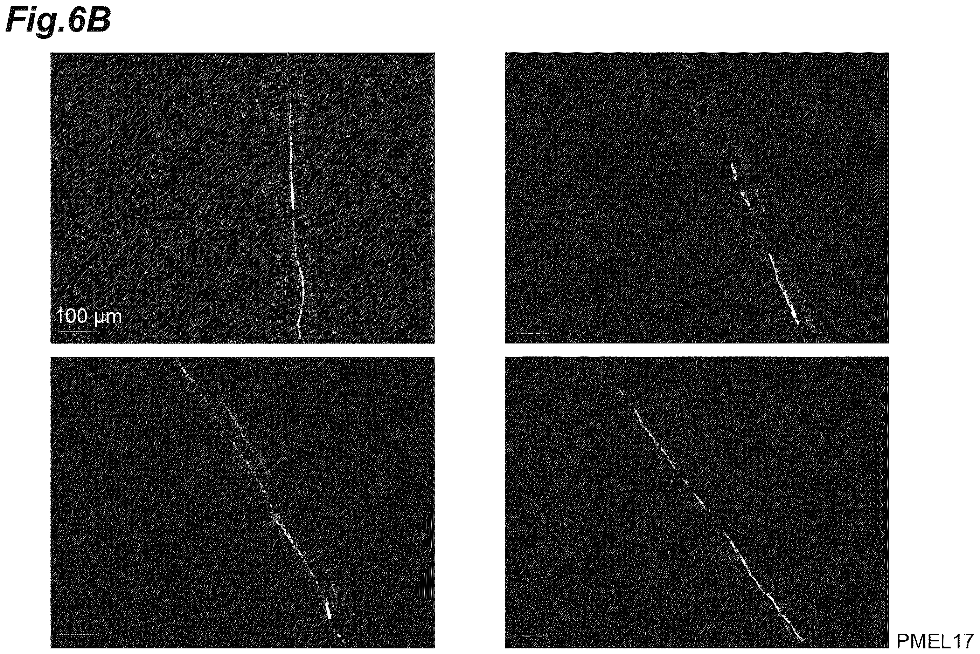

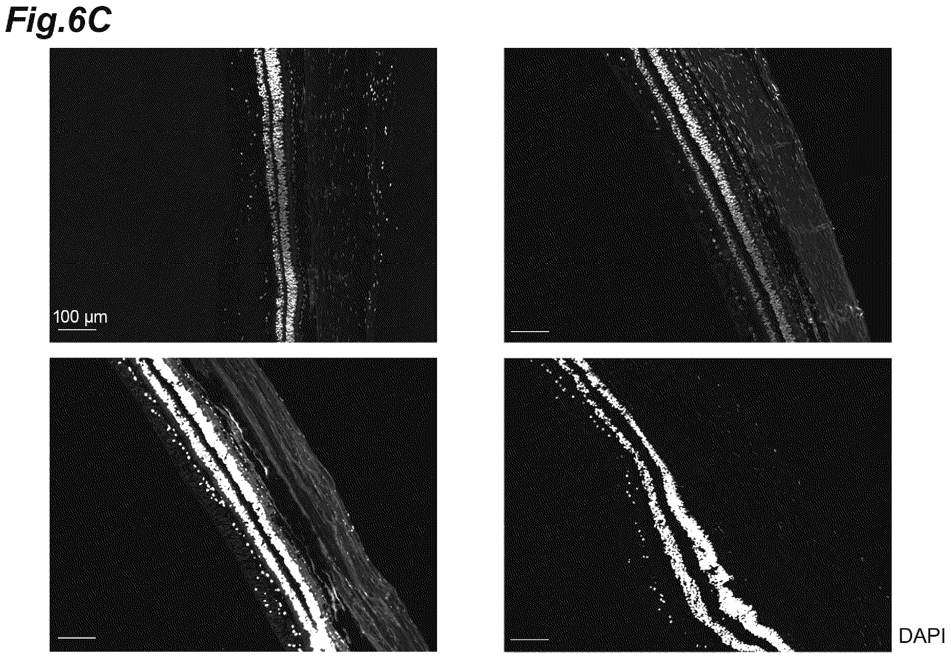

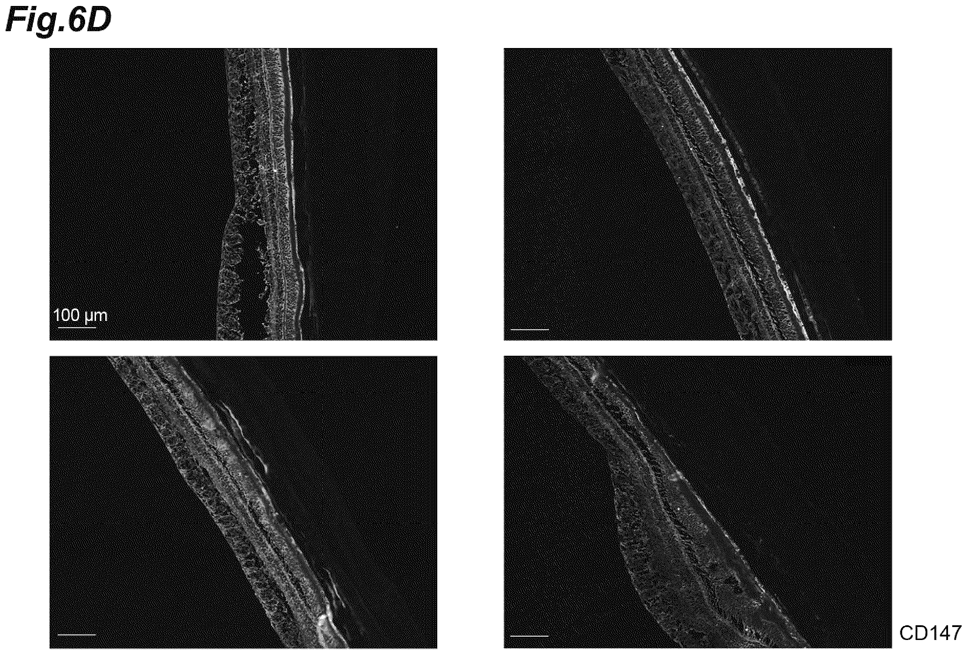

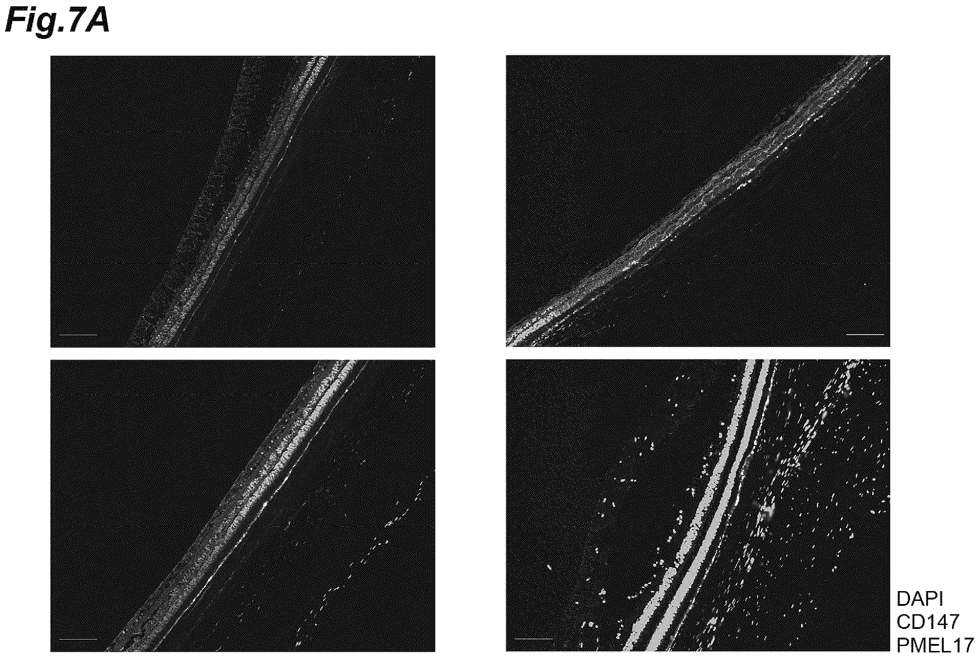

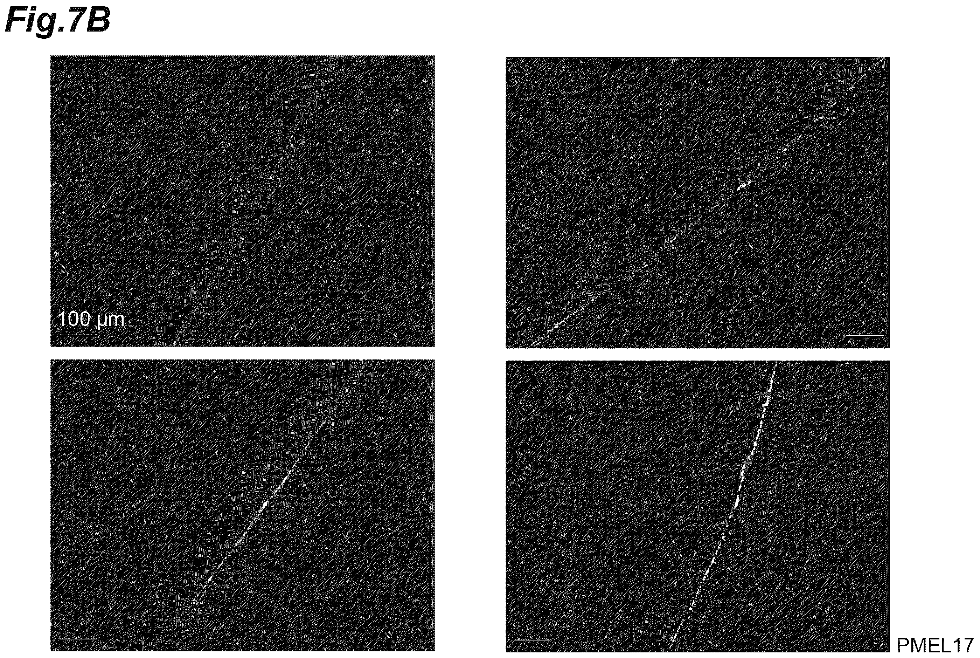

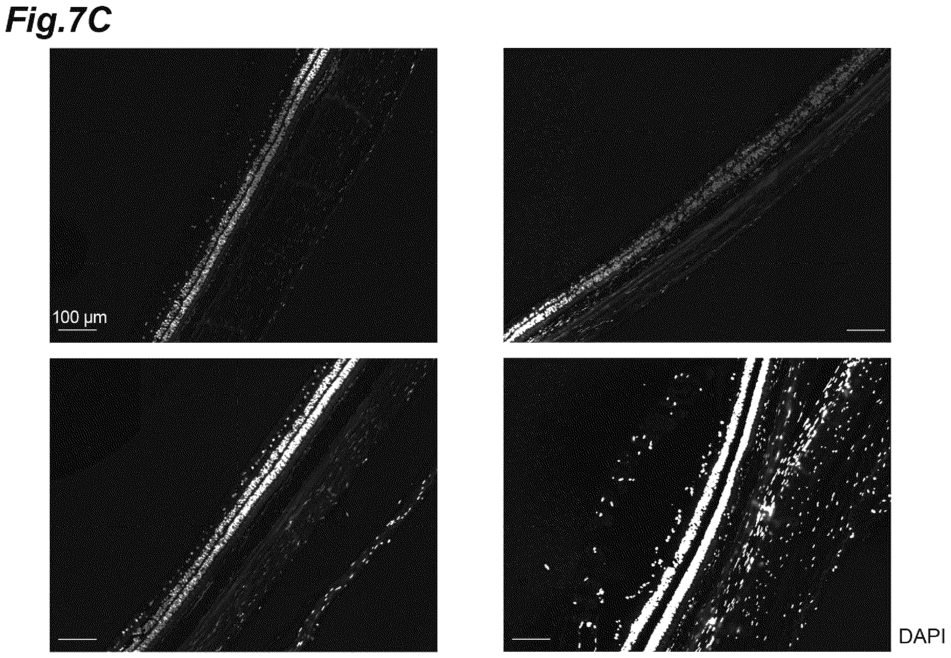

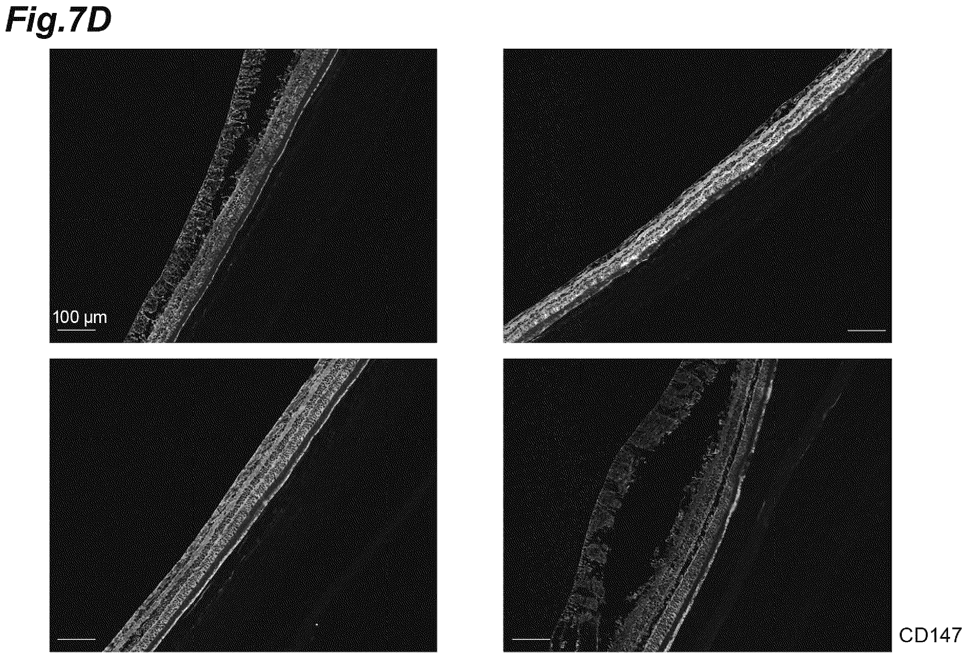

As a result of measuring the mean diameters of bleb traces using fundus images, in the HBSS(+) media (n = 4), the axial was 5.90 mm, the sagittal was 5.93 mm, and the mean basal area was 27.48 mm². On the other hand, in the PROVISC 100-fold diluted vehicle (n = 3), the axial was 4.74 mm, the sagittal was 4.30 mm, and the mean basal area was 16.01 mm². As shown in representative fluorescent immunostaining images in Figures 6A to D and 7A to D, in the fluorescent immunostaining, PMEL17-positive sites were observed in the monkey retinal pigment epithelial cell layer under any vehicle condition in all individuals, and most of the positive sites were observed in a monolayer in the monkey retinal pigment epithelial cell layer, suggesting that the administered human iPS cell-derived RPE cells were integrated and engrafted in the monkey retinal pigment epithelial cell layer. Furthermore, the engraftment grade was evaluated in the same manner as in Example 2, and the total scores in the PROVISC 100-fold diluted vehicle condition were 14 to 54, showing variation in engraftment among individuals, but the total scores in the HBSS(+) vehicle condition were 32 to 42, high in all four cases, confirming good engraftment. Therefore, both vehicle conditions showed high engraftment scores, but it was shown that the engraftment of human iPS cell-derived RPE cells was better using HBSS(+) as a vehicle compared with the PROVISC 100-fold dilution. In the HBSS(+) vehicle condition, the number of sections scored 1 or higher ranged from 14 to 19, and the horizontal engraftment range extended approximately 3.64 to 5.04 mm, whereas in the PROVISC 100-fold diluted vehicle condition, it ranged from 2.8 to 5.6 mm (number of positive sections: 11 to 21).

### <Example 4: Transplantation Experiment 3 in Cynomolgus Monkey>

Administration and evaluation were performed in the same manner as in Example 3. The subretinal injection needle used was PolyTip Tapered Cannula manufactured by MedOne Surgical, Inc., as described in Example 1. A human iPS cell-derived RPE cell suspension (1.0 × 10⁷ cells/mL, 50 µL/bleb/eye) was subretinally administered to cynomolgus monkeys using HBSS(+) as a vehicle, and ophthalmological examinations were performed over time (4 weeks and 13 weeks of observation, 3 cases each) to evaluate prolonged retinal detachment and the presence or absence of the epiretinal membrane on the macula. In addition, engraftment was evaluated by fluorescent immunostaining after the end of observation period.

The results of OCT examinations showed that no retinal detachment related to this administration method was observed at 1 week post-administration, suggesting that the bleb had resolved within less than 1 week. Furthermore, the epiretinal membrane on the macula was not observed in any individual throughout the observation period, and no membrane-like tissue on the retinal surface was observed in the histopathological evaluation by HE staining, thus suggesting that the administration vehicle and administration method were appropriate. The fluorescent immunostaining reproducibility was confirmed, and the administered human iPS cell-derived RPE cells (human PMEL17-positive cells) were integrated into the retinal pigment epithelial layer of monkeys and engrafted broadly in most animals of the 4- and 13-week observation groups, thus confirming that the engraftment of human iPS cell-derived RPE cells was maintained for 13 weeks after administration.

### <Example 5: Viscosity Measurement of Dispersion Vehicle>

The following media were prepared as dispersion media. Based on the results of Examples 1 and 2 above, the media (2) to (7) were suitable dispersion media, and among them, (3) and (6), especially (6), were preferred.

(1) Hank's balanced salt solution HBSS(+)
(2) 20-Fold diluted VISCOAT (HBSS(+):VISCOAT = 20:1)
(3) 100-Fold diluted VISCOAT (HBSS(+):VISCOAT = 100:1)
(4) 200-Fold diluted VISCOAT (HBSS(+):VISCOAT = 200:1)
(5) 20-Fold diluted PROVISC (HBSS(+):PROVISC = 20:1)
(6) 100-Fold diluted PROVISC (HBSS(+):PROVISC = 100:1)
(7) 200-Fold diluted PROVISC (HBSS(+):PROVISC = 200:1)

The viscosities of the media (1) to (7) above were measured using a viscosity measuring device under the conditions of 25°C and a shear rate of 1/s = 2. The results are shown in Table 5.

**[Table 5]**

| Vehicle | Viscosity (mPa·s) | Concentration of sodium hyaluronate (w/v) | Concentration of chondroitin sulfate sodium (w/v) |
|---|---|---|---|
| (1)HBSS(+) | 0.829 | N.A | N.A |
| (2) HBSS(+)/VISCOAT (20:1) | 4.38 | 0.15% | 0.20% |
| (3) HBSS(+)/VISCOAT (100:1) | 2.23 | 0.03% | 0.04% |
| (4) HBSS(+)/VISCOAT (200:1) | 1.17 | 0.015% | 0.02% |
| (5) HBSS(+)/PROVISC (20:1) | 5.78 | 0.05% | N.A |
| (6) HBSS(+)/PROVISC (100:1) | 2.13 | 0.01% | N.A |
| (7) HBSS(+)/PROVISC (200:1) | 1.25 | 0.005% | N.A |

| | | | |
|---|---|---|---|
| N.A.: substantially free | | | |

The correlation between rate of shear (shear rate) and viscosity of the media (1) to (7) was measured under the condition of 25°C using a viscosity measuring device. The results are shown in Table 6. As a result, it was found that among the media (1) to (7), the amount of change in viscosity of the media (3) and (6) was larger than that of the other media.

**[Table 6]**

| Vehicle | Viscosity at each shear rate (1/s) (mPa·s) | | | | | Viscosity difference* (mPa·s) |
|---|---|---|---|---|---|---|
| | 1 | 2 | 10 | 100 | 1000 | |
| (1)HBSS(+) | 0.981 | 0.829 | 0.865 | 0.890 | 0.909 | 0.12 |
| (2) HBSS(+)/VISCOAT (20:1) | 4.45 | 4.38 | 4.37 | 4.36 | 3.97 | 0.08 |
| (3) HBSS(+)/VISCOAT (100:1) | 2.68 | 2.23 | 1.96 | 1.71 | 1.31 | 0.72 |
| (4) HBSS(+)/VISCOAT (200:1) | 1.35 | 1.17 | 1.09 | 1.08 | 1.07 | 0.26 |
| (5) HBSS(+)/PROVISC (20:1) | 5.87 | 5.78 | 5.63 | 4.66 | 3.07 | 0.24 |
| (6) HBSS(+)/PROVISC (100:1) | 3.13 | 2.13 | 1.89 | 1.66 | 1.25 | 1.24 |
| (7) HBSS(+)/PROVISC (200:1) | 1.30 | 1.25 | 1.16 | 1.14 | 1.07 | 0.14 |

| | | | | | | |
|---|---|---|---|---|---|---|
| *Absolute value of difference in viscosity (mPa·s) between viscosity at shear rate 1 (1/s) and viscosity at shear rate 10 (1/s) | | | | | | |

### <Example 6: Evaluation of Efficacy Using RPE Dysfunction Model Rat>

The nonclinical efficacy of human iPS cell-derived RPE cells was examined by a method of subretinal administration into pigmented RCS rats. Since RCS rats lack the phagocytic ability of photoreceptor outer segments, which RPE cells originally possess, due to a mutation in the MERTK gene, resulting in secondary degeneration of photoreceptors due to abnormal accumulation of outer segments, the RCS rats are widely used as a model of RPE dysfunction to evaluate the pharmacological efficacy of RPE cell replacement therapy (Hum Mol Genet. 2000; 9(4): 645-51., Stem Cells Int. 2017; 2017:9428176.).

To the left eyes of pigmented RCS rats (4-week-old) immunosuppressed with cyclosporine in drinking water, 1 × 10⁵ cells of human iPS cell-derived RPE cells were subretinally administered. After administration, the visual function of the administered eyes was evaluated by electroretinography (ERG), visual evoked potential (VEP), and optokinetic tracking (OKT) tests over time. The engraftment and function of human iPS cell-derived RPE cells were evaluated by fluorescent immunostaining of the enucleated eyes 2 months after administration.

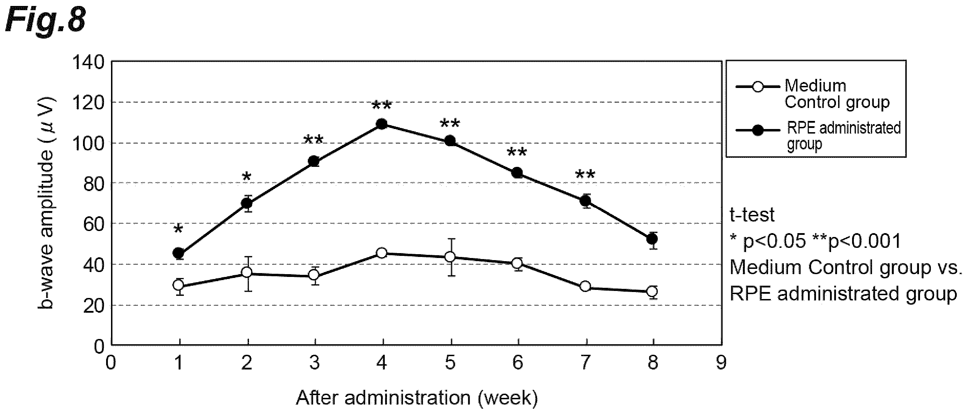

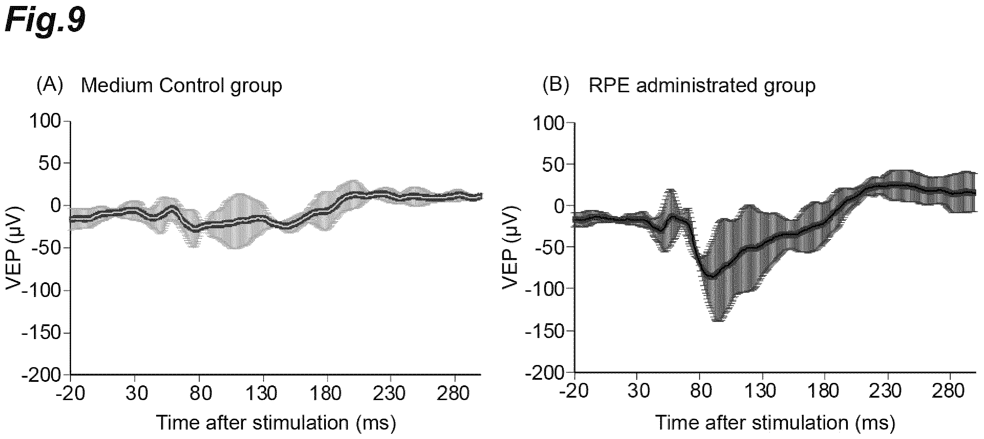

Comparison of ERG b-wave amplitude revealed loss of response from 2 weeks after administration in the vehicle control group, but significant recovery was observed up to 7 weeks after administration in the administered group. Figure 8 shows the results. The VEP tended to show a greater response in the administered group than in the vehicle control group at 1 month after administration. Figure 9 shows the results.

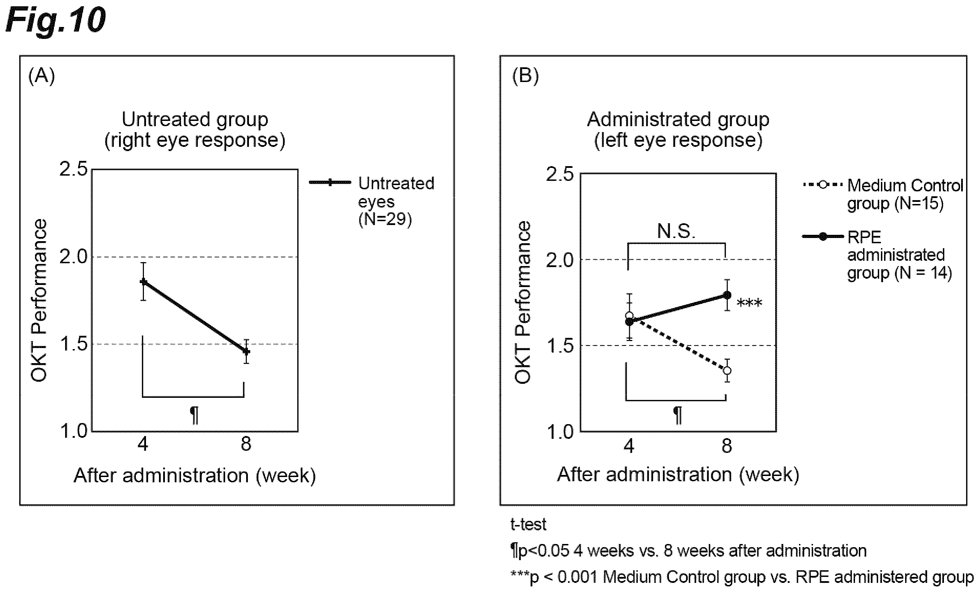

In the OKT test, performance values were calculated as relative values of Correct to Incorrect, where Correct was defined as head-tracking behavior observed in the same direction as the rotation of the striped pattern and Incorrect as head-tracking behavior observed in the opposite direction. Figure 10 shows the results.

In the vehicle control group and the untreated group (right eyes), a significant decline in visual function was observed over time (Figures 10(A) and (B)). On the other hand, in the human iPS cell-derived RPE cell administered group (left eyes), no significant decline in visual function was observed up to 2 months after administration (Figure 10(B)). At 2 months after administration, significant retention of visual function was observed in the human iPS cell-derived RPE cell administered group as compared to the vehicle control group (Figure 10(B)).

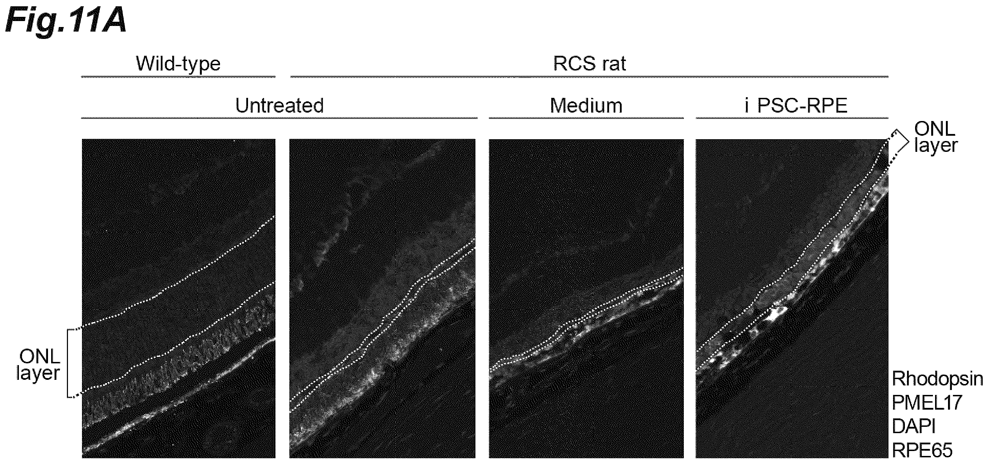

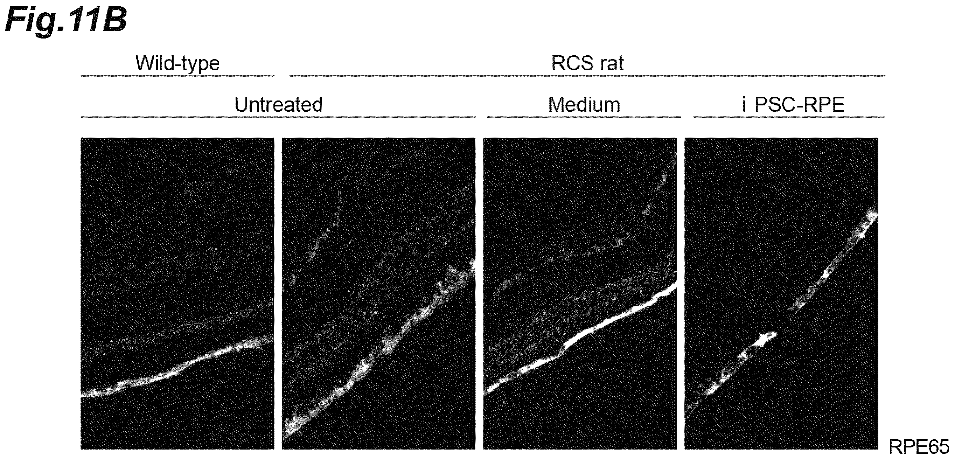

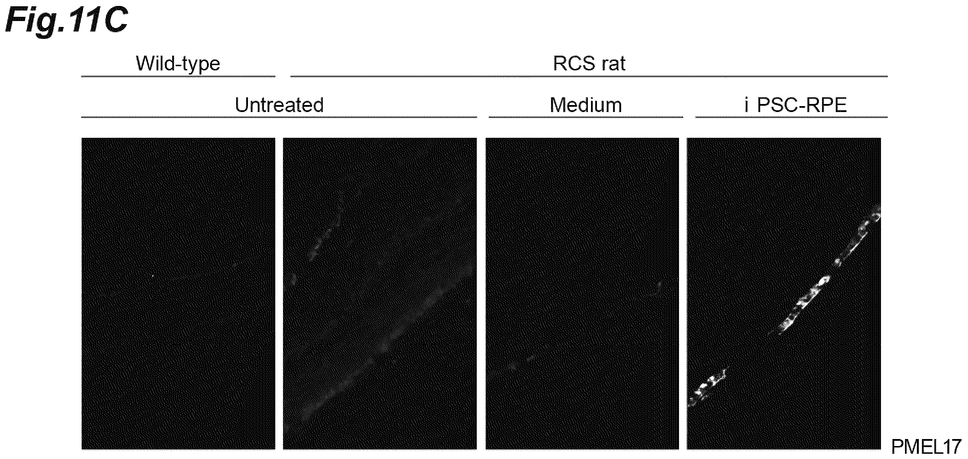

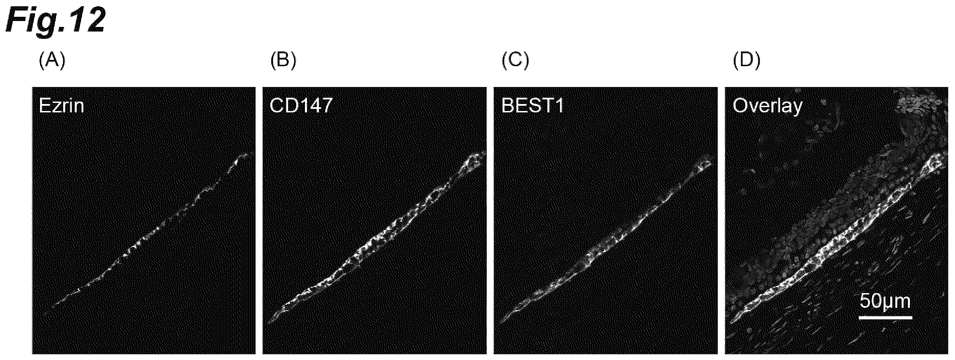

The engraftment of human iPS cell-derived RPE cells at 2 months after administration was evaluated by fluorescent immunostaining. Figures 11A to 11C and 12 shows the results. In the human iPS cell-derived RPE cell administered groups ("iPSC-RPE" in Figures 11A to C), RPE cells stained with antibodies specifically recognizing the human-type PMEL17 engrafted integrally or overlapped with the RPE layer (RPE65-positive portion) of RCS rats (Figures 11A to C). As a result of performing fluorescent immunostaining for Ezrin expressed in the apical region, CD147, and BEST 1 expressed in the basolateral region along with the maturation of RPE, the respective expression was observed locally in the cells, demonstrating that human iPS cell-derived RPE cells matured and engrafted with physiological cell polarity after transplantation (Figure 12).

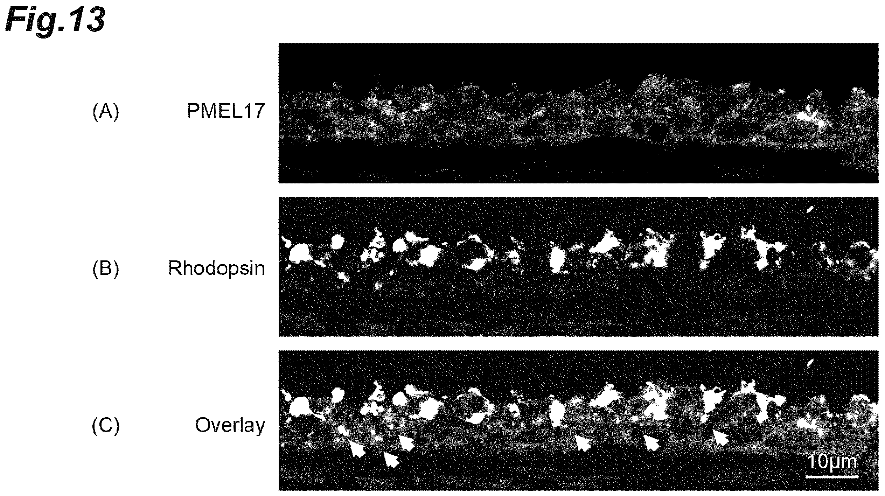

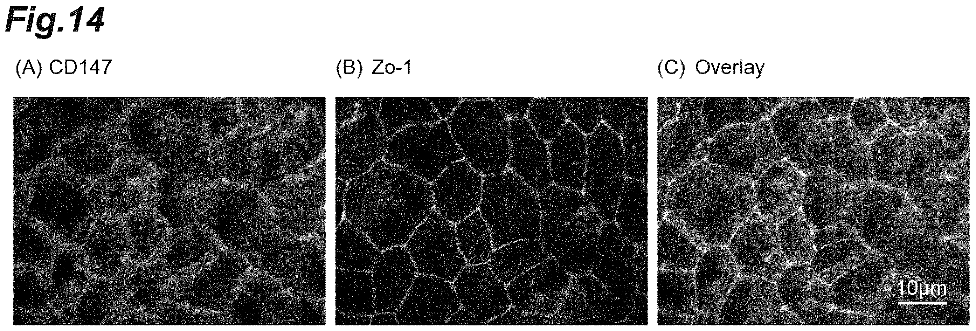

The phagocytic ability and barrier function of human iPS cell-derived RPE cells after transplantation were also evaluated by fluorescent immunostaining. Vesicles containing rat photoreceptor outer segments stained with anti-Rhodopsin antibody were observed inside human iPS cell-derived RPE cells stained with anti-human PMEL17 antibody (Figure 13(C), indicated by arrows), confirming the phagocytic ability of transplanted human iPS cell-derived RPE cells. Retinal whole-mount staining showed the expression of Zo-1, a component of tight junctions, overlapping with human iPS cell-derived RPE cells stained with anti-CD147 antibody, suggesting that human iPS cell-derived RPE cells were engrafted and exerted barrier function (Figure 14).

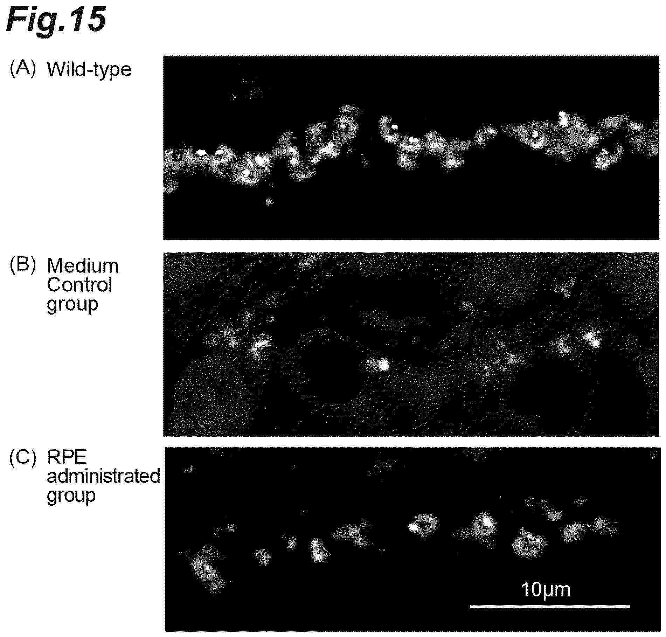

Photoreceptor protection was evaluated by the persistence of the outer nuclear layer (ONL), which is composed of photoreceptor somata. In the non-transplanted group and the vehicle control group, the ONL (indicated by the dotted line in Figure 11A) was completely lost or only partially remained in a layer consisting of 1 or 2 cells, but in the human iPS cell-derived RPE cell administered group, a significantly greater amount of ONL remained in contact with the engraftment site than in the vehicle control group (Figure 11A). The synapses between the photoreceptors and bipolar cells in the outer plexiform layer (OPL) were detected by fluorescent immunostaining for CtBP2, which expressed on the presynaptic terminal, and mGluR6, a postsynaptic receptor. As a result, most of the synapses were lost in the vehicle control group, whereas a certain number of residual synapses were observed in the RPE-administered group (Figure 15). These results showed that human iPS cell-derived RPE cells engrafted into the subretinal space, matured and became functional within 2 months after administration, exerted an inhibitory effect on photoreceptor degeneration in RCS rats, had a protective effect on the retina including photoreceptors, and had the effect of preserving visual function.

### <Example 7: Evaluation of RPE Cell Adhesiveness with and without Laminin Coating>

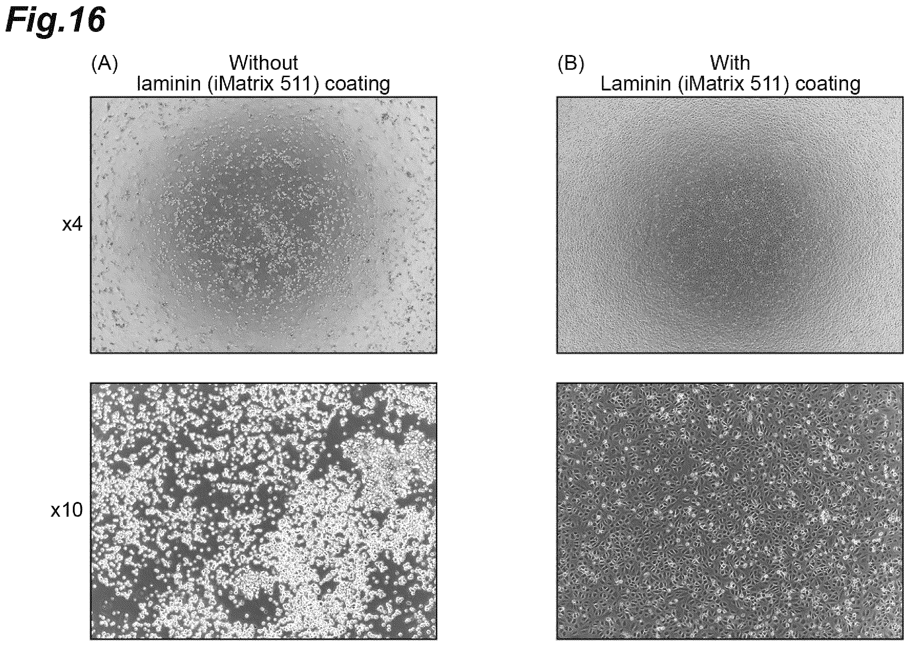

Adhesiveness of RPE cells to wells coated with iMatrix511(0.5 µg/cm²), an E8 fragment of laminin 511, as a substrate having properties similar to those of components forming the Bruch's membrane and to uncoated wells was evaluated. Human iPS cell-derived RPE cells were seeded at 2.5 × 10⁷ cells/well in a 24-well plate and cultured in an RPE maintenance medium (a mixed medium of 355 mL of DMEM, 150 mL of F-12 HAM, 5 mL of L-Gln, and 10 mL of B-27). As a result of observation on the next day of seeding, adhesion of RPE cells was observed in the wells coated with iMatrix511 (Figure 16(B)), but not in the uncoated wells (Figure 16(A)). Good adhesion of RPE cells to the laminin-coated substrate was demonstrated.

### <Example 8: Examination of Usefulness of RPE Cell Suspension Using In Vitro Retinal Pigment Epithelium Tear Model>

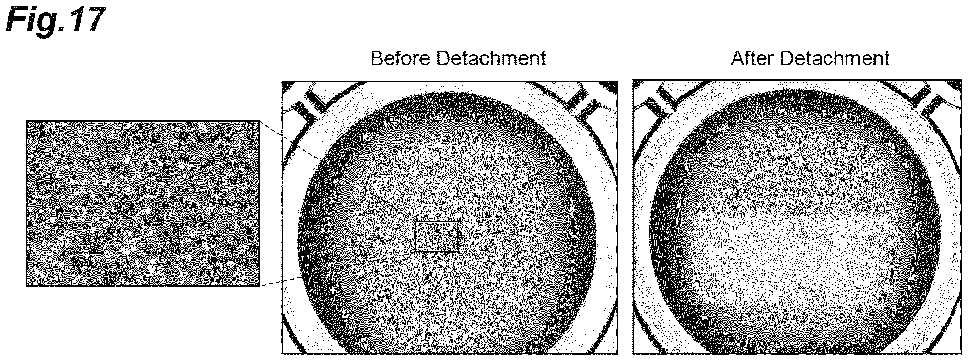

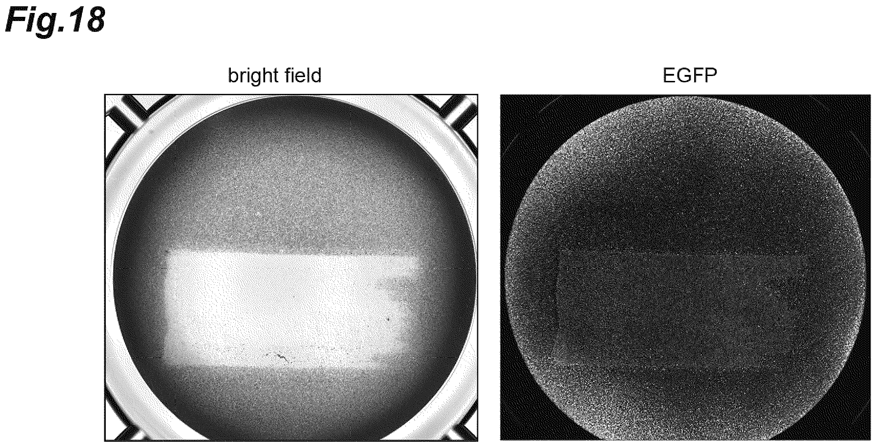

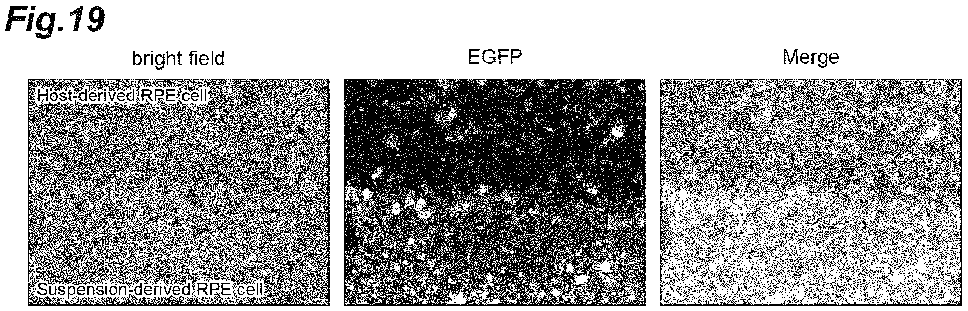

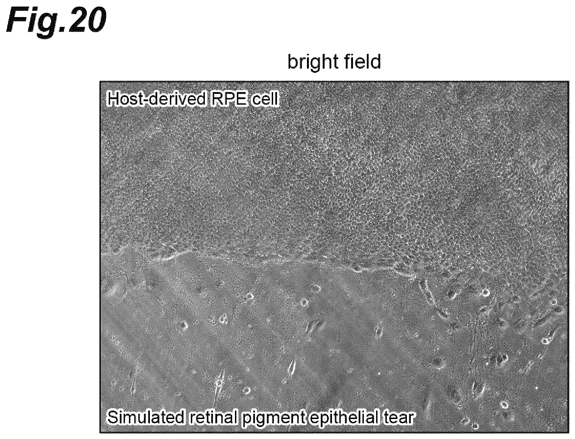

Human iPS cell-derived RPE cells were seeded in 24-well plates coated with iMatrix511(0.5 µg/cm²), cultured, and then RPE sheets were formed. To inhibit cell proliferation of RPE sheets, RPE sheets were treated with Mitomycin C at 6 µg/mL. After 1 day of culture, a part of the RPE sheet (approximately 5 mm × 10 mm) was detached with a cell scraper to form simulated retinal pigment epithelial tears (Figure 17). To mimic well-preserved Bruch's membrane at the detached site, recoating with iMatrix511 was performed. Subsequently, a human iPS cell-derived RPE cell suspension expressing EGFP was seeded at 1 × 10⁶ cells/well as a simulated transplantation RPE cell suspension. The number of seeded cells was set based on the number of cells per area, considering the whole well as the bleb at the time of transplantation. The day after seeding the simulated transplantation RPE cell suspension, EGFP-expressing human iPS cell-derived RPE cells adhered to the entire simulated retinal pigment epithelial tears (Figure 18). Six days after seeding, it was confirmed that suspension-derived EGFP-expressing human iPS cell-derived RPE cells completely filled the boundary with the host-side RPE cells to form a single RPE sheet (Figure 19). On the other hand, the detached part of the untreated simulated retinal pigment epithelial tears remained as they were formed (Figure 20).

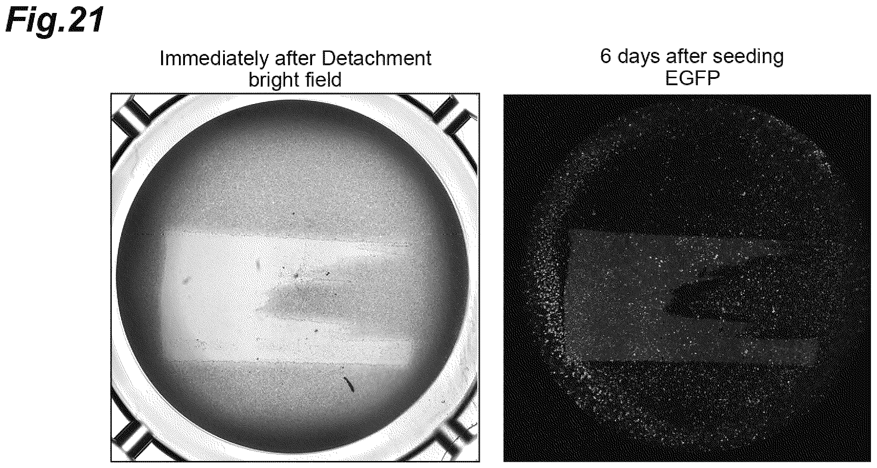

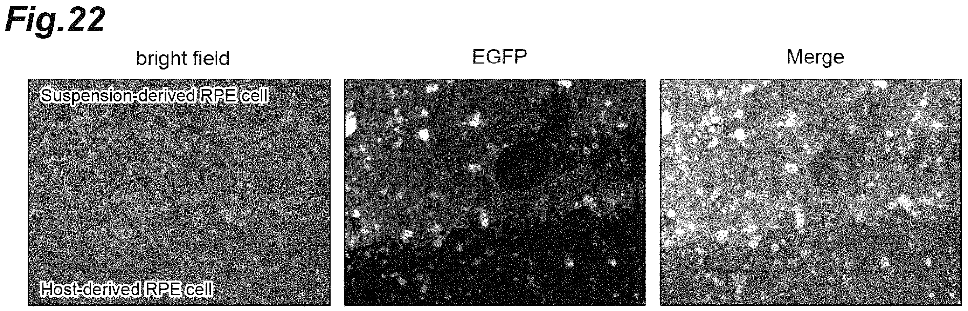

Since the classic shape of retinal pigment epithelial tears is a crescent-shaped tear, but various shapes also exist, the usefulness of RPE cell suspensions for simulated retinal pigment epithelial tears with complex shapes was examined next by a method using simulated retinal pigment epithelial tears as well. As a result, it was confirmed that suspension-derived RPE cells could engraft at the detachment site without gaps and restore a single RPE sheet even for simulated retinal pigment epithelial tears with complex shapes (Figures 21 and 22). In contrast to typical RPE sheet formulations, in which incomplete coverage of the detached area or overlapping with normal RPE cells is expected, RPE cell suspensions showed the potential to rapidly restore the host RPE sheet in retinal pigment epithelial tears of various shapes without incomplete coverage.

### <Example 9: Phase I/II Study of iPS Cell-Derived RPE Cell Product in Patient Having Retinal Pigment Epithelial Tear>

Objective: To evaluate the safety and efficacy of subretinal administration of a suspension of an iPS cell-derived RPE cell product in patients with retinal pigment epithelial tears (RPE tears) in a randomized, unmasked, multicenter study. The efficacy of the suspension of iPS cell-derived RPE cells, including visual acuity and retinal sensitivity is evaluated.

### Design

It consists of two parts, Part 1 and Part 2, and starts with Part 1.
Part 1: HLA mismatched subjects receive a suspension of iPS cell-derived RPE cell products.
Part 2: The subjects are randomly assigned to an administration group or an observation group, and the administration group receives a suspension of iPS cell-derived RPE cell product. The observation group receives a suspension of iPS cell-derived RPE cell product after completion of 48 weeks of observation.
Observation period: Both consist of two periods: a treatment period (48 weeks) and a continued treatment period (48 weeks).
Dose: A single dose of 50 µL of a 1.0 × 10⁷ cells / mL cell suspension is administered subretinally into the eye to be treated. However, at the time of cell suspension injection, the standard practice is for the operator to administer a dose covering the major axis of the RPE tear, and administration is completed when a bleb of that size has been created. Therefore, the dose of cell suspension is a maximum of 50 µL (5 × 10⁵ cells), but the dose might be reduced as appropriate.
Concomitant medications: Immunosuppressive agents such as triamcinolone acetonide, cyclosporine, and prednisolone are administered to suppress immune rejection associated with cell transplantation or to suppress postoperative inflammation.
Target number of cases: 21 in total
Part 1: 1 HLA mismatched subject
Part 2: 10 randomized subjects per group (20 in total)

### Target Patient:

### Main selection criteria:

1) Patients in whom, an RPE tear (with a long diameter greater than one disc diameter) secondary to age-related macular degeneration (AMD) or other retinal diseases, or idiopathic, has been identified in the macular region of the eye to be treated by fundus photography (including autofluorescence) and OCT
2) Patients within 6 months after RPE tear was confirmed at the time of obtaining informed consent
3) Patients whose BCVA according to the ETDRS chart in the eye to be treated is 24 letters or more and 78 letters or less at screening and baseline
4) (Part 1 only) HLA mismatched patient

### Main exclusion criteria:

1) Patients with retinal disease that would prevent evaluation
2) Patients with poorly controlled systemic disease

Primary endpoint: adverse events
Secondary endpoints: visual acuity (ETDRS chart), retinal sensitivity (microperimetry, Humphrey visual field test), OCT examination, Visual Function Questionnaire (VFQ-25), Euro-QOL5 Dimension-5 Level (EQ-5D-5L), Health Utilities IndexMark3 (HUI3)

### <Example 10: Evaluation of Efficacy Using Retinal Pigment Epithelium Degeneration Model Rat>

The nonclinical efficacy of human iPS cell-derived RPE cells was examined by a method of administrating human iPS cell-derived RPE cells into the subretinal space of a retinal pigment epithelium degeneration model (sodium iodate-induced model) rat. It is known that systemic administration of sodium iodate to rodents causes specific damage to RPE cells, which are widely used as retinal pigment epithelium degeneration models (J Photochem Photobiol B. 2019 Jul:196:111514.).

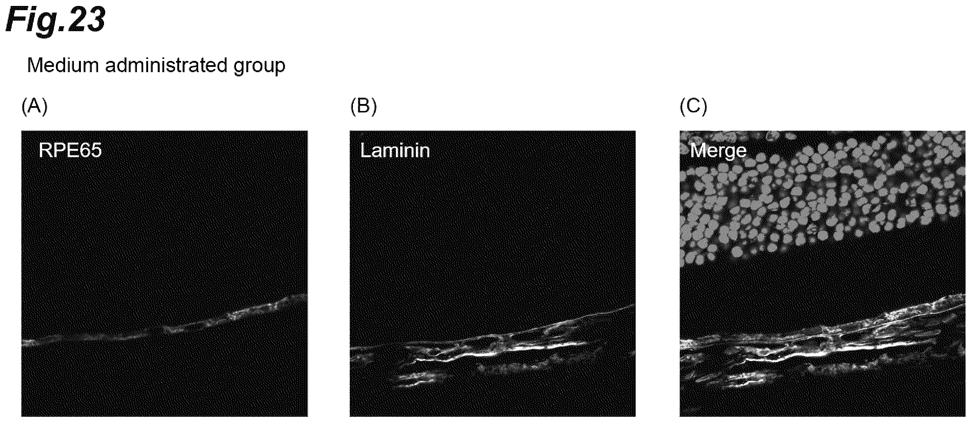

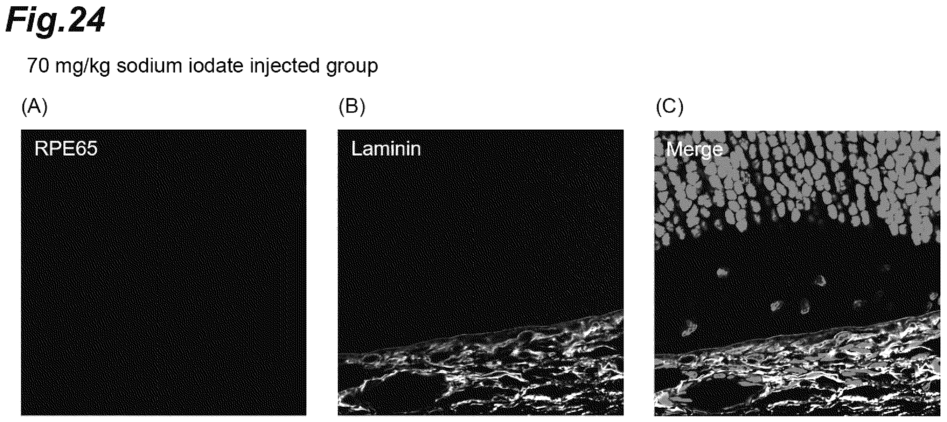

Sodium iodate was intraperitoneally administered to an immunodeficiency model F344/NJcl nude rat (7-week-old) at 0 (administration vehicle only) or 70 mg/kg (dose volume: 0.5 mL/kg), and the RPE and basement membrane of the rat were evaluated by fluorescent immunostaining using the enucleated eye 7 days later. Figures 23(A) to (C) and Figures 24(A) to (C) show the results. Since RPE65, an RPE cell marker, was negative in the 70 mg/kg sodium iodate administered group, it was confirmed that rat RPE cell-specific damage occurred (Figure 24(A)). In addition, as a result of staining of laminin, a constituent of the rat basement membrane, it was revealed that laminin was expressed and the basement membrane was maintained in both the vehicle administered group and the 70 mg/kg sodium iodate administered group (Figure 23(B) and Figure 24 (B)). In other words, in the sodium iodate-induced model rat 7 days after the administration of sodium iodate, RPE degeneration progressed while the basement membrane was maintained, suggesting that the pathological condition was similar to that of a retina with retinal pigment epithelial tears.

Sodium iodate was intraperitoneally administered to an immunodeficiency model F344/NJcl nude rat (7- or 9-week-old) at 0 or 70 mg/kg (dose volume: 0. 5 mL/kg). Seven days later, 1 × 10⁵ cells of human iPS cell-derived RPE cells were subretinally administered into the left or right eye. Electrophysiological function was evaluated by an electroretinography (ERG) test about 1 month after administration, and fundus images were confirmed by fundus camera photography about 2 months after administration, and the engraftment area of human iPS cell-derived RPE cells was evaluated. Then, the engraftment of human iPS cell-derived RPE cells and the host retina were evaluated by fluorescent immunostaining of the enucleated eye about 2 months after administration.

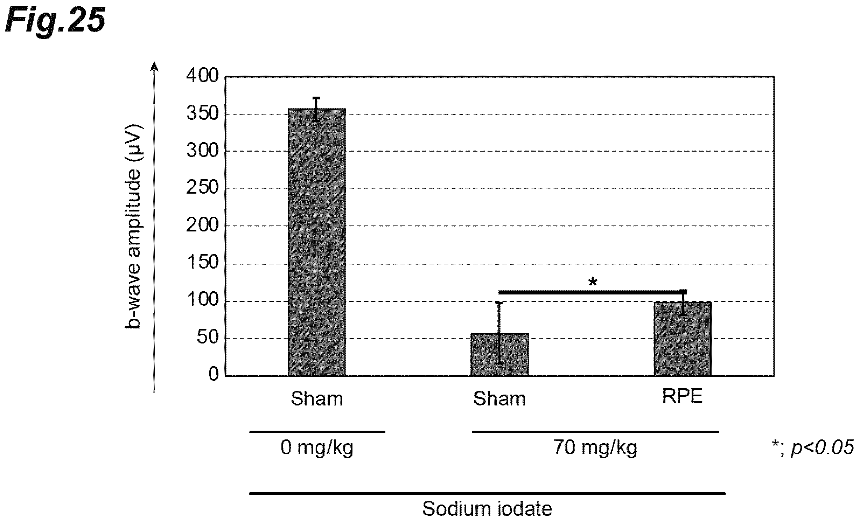

Electrophysiological function at approximately 1 month after administration was evaluated by the ERG test. Rats were dark-adapted for at least 3 hours and then ERG was measured under light stimulation conditions of 10 cd × s/m². Figure 25 shows the results. The b-wave amplitude was about 350 µV in the vehicle administered group (left side of Figure 25, Sham) after administration of 0 mg/kg sodium iodate as a control, and a significant decrease in the b-wave amplitude was observed in the vehicle administered group (the center of Figure 25, Sham) after administration of 70 mg/kg sodium iodate, resulting in a decrease in electrophysiological function that was considered to be caused by impairment of rat RPE cells. On the other hand, a significant increase in b-wave amplitude was observed in the RPE cell administered group (right side of Figure 25, RPE) after administration of 70 mg/kg sodium iodate compared with the vehicle administered group after administration of 70 mg/kg sodium iodate. In addition, a similar ERG test was performed approximately 2 months after administration, and as a result, a significant increase in b-wave amplitude was observed. These results demonstrated that administered human RPE cells engrafted into the rat subretina and had an inhibitory effect on the decrease in electrophysiological function.

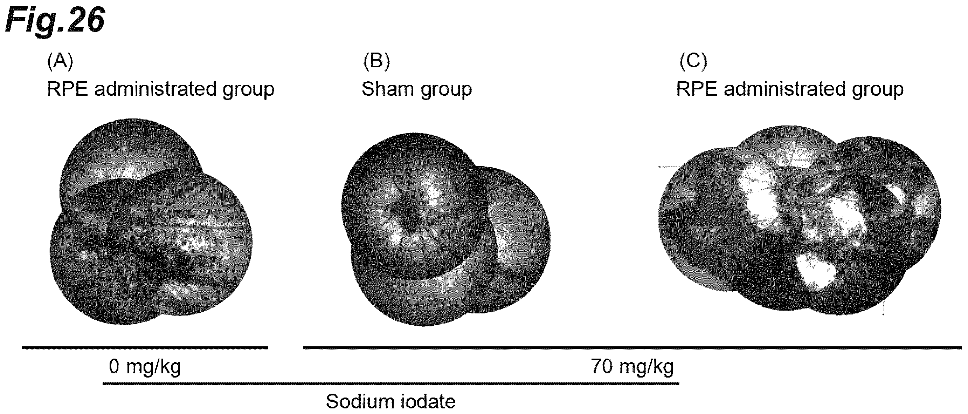

About two months after the administration, fundus images were captured by fundus camera imaging to evaluate an engrafted region of the human iPS cell-derived RPE cells. Figures 26(A) to (C) show the results. Engrafted human RPE cells were recognized as black areas in the fundus by melanin deposition. Since nude rats had albino eyes, there was no melanin deposition in RPE cells. Therefore, the black areas observed were determined to be melanin deposition in the transplanted human RPE cells. The engrafted region of human RPE cells in the sodium iodate administered group (Figure 26(C)) was wider than the engrafted region of human RPE cells in the vehicle administered control group (Figure 26(A)). On the other hand, no black region was observed in the vehicle administered group after administration of 70 mg/kg sodium iodate (Figure 26(B)).

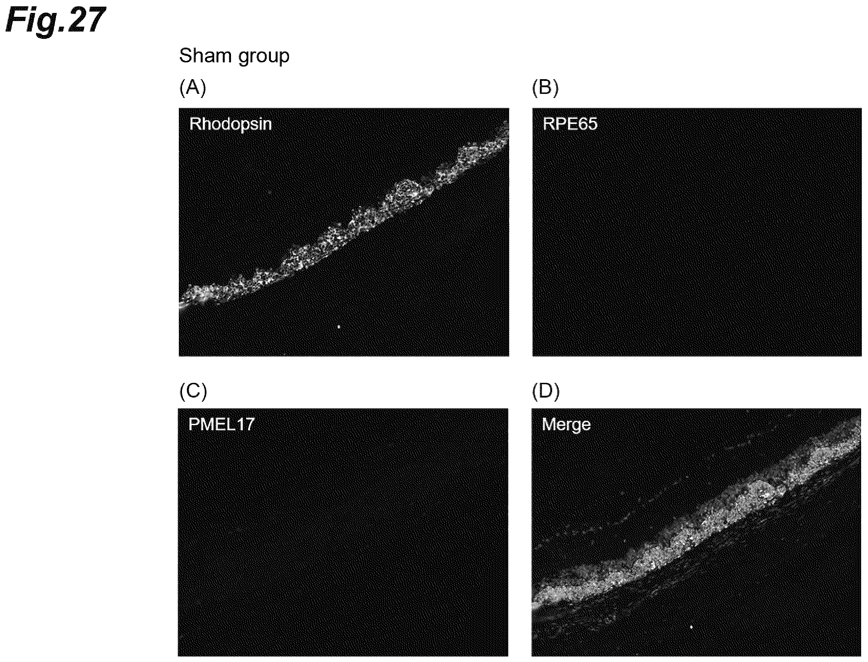

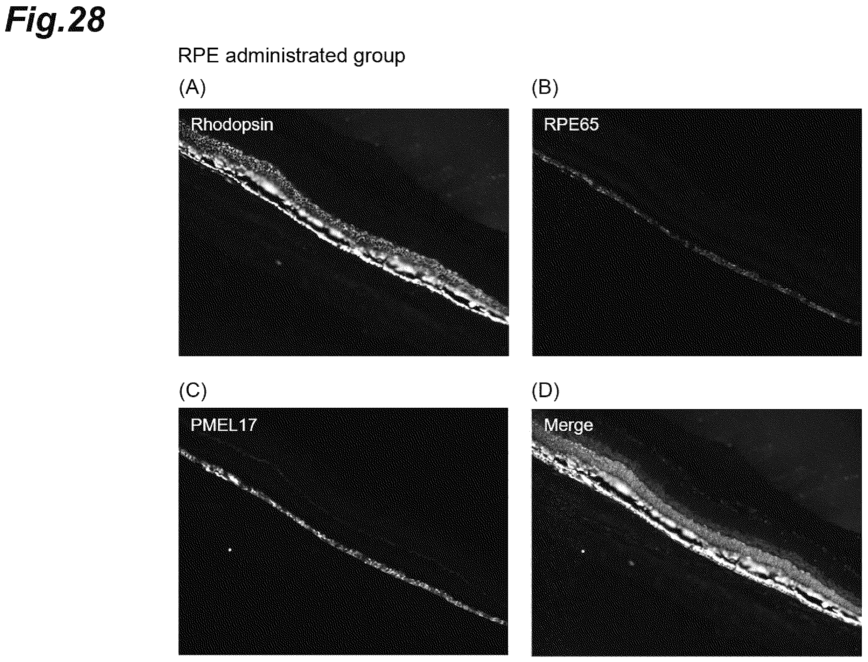

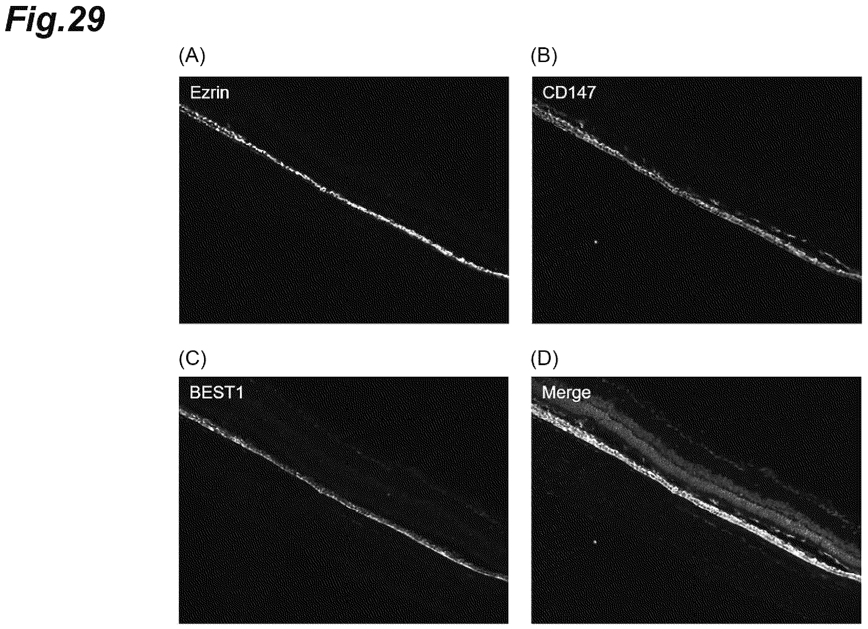

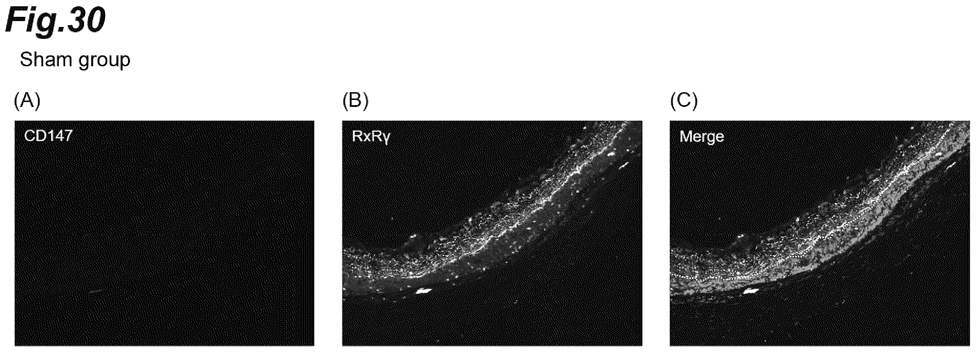

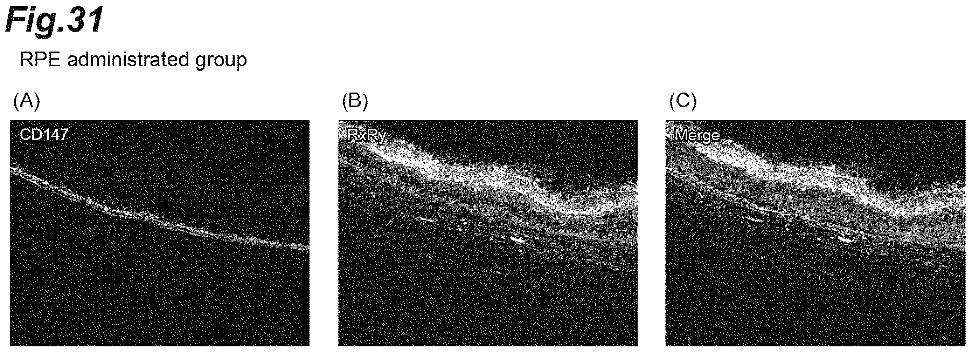

The engraftment of human iPS cell-derived RPE cells and the rat retina were evaluated by fluorescent immunostaining of the enucleated eyes approximately 2 months after administration. Figures 27 to 31 show the results. In the group receiving sodium iodate followed by subretinal administration of the vehicle (Sham group), loss of rat RPE cells stained with an antibody specifically recognizing rat/human RPE65 was observed (Figure 27 (B)). On the other hand, in the human iPS cell-derived RPE cell administered group (RPE administered group), human RPE cells stained with an antibody specifically recognizing human PMEL17 were mostly engrafted in a single layer of the rat RPE layer (Figure 28 (C)). In the same administration group, as a result of performing fluorescent immunostaining for Ezrin expressed in the apical region, CD147, and BEST 1 expressed in the basolateral region along with the maturation of RPE, the expression was observed in the respective cellular localizations, demonstrating that human iPS cell-derived RPE cells matured and engrafted with physiological polarity after transplantation (Figures 29(A) to (D)). The evaluation of the rat retina was performed using the shape of the ONL and the localization of the rat cone cells as indices. In the group receiving sodium iodate followed by subretinal administration of the vehicle (Sham group), it was suggested that the shape of the ONL (Figure 30(C)) was disturbed due to the influence of the disorder of the RPE cells, and the localization of the rat cone cells denoted by RxRy specifically stained for rat cone cells was also disturbed (Figure 30(B)). On the other hand, in the human iPS cell-derived RPE cell administered group (RPE administered group), the shape of the ONL (Figure 31(C)) was maintained in a state close to normal in the vicinity of the engrafted region of the human RPE cells stained with CD147, and rat cone cells denoted by RxRy were also observed to be present in an aligned manner (Figure 31(B)). From these results, it was shown that when human iPS cell-derived RPE cells were administered subretinally to a retinal pigment epithelial degeneration model (sodium iodate-induced model) rat, the human iPS cell-derived RPE cells were widely engrafted in the rat RPE layer and had an inhibitory effect on a decrease in electrophysiological function until about 2 months after the administration, further matured and had physiological polarity about 2 months after the administration, and had an effect of maintaining the shape of the rat retina.

## Claims

1. A therapeutic agent for a retinal pigment epithelial tear in a human subject, consisting of a suspension of dispersed human retinal pigment epithelial cells.

2. The therapeutic agent according to claim 1, wherein a viscosity of a vehicle used for dispersing the human retinal pigment epithelial cells is 4 mPa·s or less at a shear rate of 2 (1/s) at 25°C.

3. The therapeutic agent according to claim 1 or 2, wherein a concentration of the human retinal pigment epithelial cells in the suspension is 0.1 × 10⁷ to 1 × 10⁷ cells/mL.

4. The therapeutic agent according to any one of claims 1 to 3, wherein a dose volume of the therapeutic agent is 20 to 50 µL.

5. The therapeutic agent according to any one of claims 1 to 4, which is administered into a region surrounding the retinal pigment epithelial tear through a port of about 0.4 mm to about 0.65 mm on the sclera of the human subject while forming a bleb.

6. The therapeutic agent according to claim 5, wherein the bleb is formed to cover a major axis of the retinal pigment epithelial tear.

7. The therapeutic agent according to claim 5 or 6, for use in treatment comprising replacing the vitreous cavity of the human subject with air after administration of the therapeutic agent.

8. The therapeutic agent according to any one of claims 2 to 7, wherein after administration of the therapeutic agent to the human subject, no formation of the epiretinal membrane is observed or formation of the epiretinal membrane is reduced to some extent.

9. The therapeutic agent according to any one of claims 1 to 8, wherein the retinal pigment epithelial tear is in a state where a retinal pigment epithelial cell layer of the human subject is partially ruptured, and Bruch's membrane exposed without being covered with retinal pigment epithelial cells of the human subject remains as a basement membrane.

10. The therapeutic agent according to claim 9, wherein after administration of the therapeutic agent to the human subject, the human retinal pigment epithelial cells become polarized and cover the exposed Bruch's membrane.

11. The therapeutic agent according to any one of claims 1 to 10, wherein the human retinal pigment epithelial cells are derived from *in vitro* differentiation of human pluripotent stem cells.

12. The therapeutic agent according to any one of claims 1 to 11, wherein the human subject has a human leukocyte antigen (HLA) type that matches with the human retinal pigment epithelial cells.

13. The therapeutic agent according to any one of claims 1 to 12, which is administered in combination with at least one immunosuppressive agent.

14. The therapeutic agent according to claim 13, wherein the at least one immunosuppressive agent is at least one selected from the group consisting of triamcinolone acetonide, cyclosporine, tacrolimus, prednisolone and dexamethasone.
